(19) Europäisches Patentamt
European Patent Office
Office européen des brevets

(11) Publication number: **0 316 967 B1**

(12) # EUROPEAN PATENT SPECIFICATION

(45) Date of publication of patent specification: **09.09.92**

(51) Int. Cl.5: **C07D 233/91**, A61K 31/415

(21) Application number: **88121308.6**

(22) Date of filing: **10.06.88**

(60) Publication number of the earlier application in accordance with Art.76 EPC: **0 294 847**

The file contains technical information submitted after the application was filed and not included in this specification

(54) **Fluorine-containing nitroimidazole derivatives and radiosensitizer comprising the same.**

(30) Priority: **24.06.87 JP 156787/87**
**29.01.88 JP 20456/88**

(43) Date of publication of application:
**24.05.89 Bulletin 89/21**

(45) Publication of the grant of the patent:
**09.09.92 Bulletin 92/37**

(84) Designated Contracting States:
**AT BE CH DE ES FR GB GR IT LI LU NL SE**

(56) References cited:
**EP-A- 0 000 928**

**CHEMICAL ABSTRACTS, vol. 89, no. 23, 4th December 1978, page 46, abstract no. 191005a**

**CHEMICAL ABSTRACTS, vol. 89, no. 23, 4th December 1978, page 46, abstract no. 191006b**

(73) Proprietor: **Nishijima, Yasunori**
**President Kyoto University Honmachi,**
**Yoshida Sakyo-ku**
**Kyoto-shi Kyoto-fu(JP)**

Proprietor: **DAIKIN INDUSTRIES, LIMITED**
**Umeda Center Building, 4-12, Nakazaki-nishi**
**2-chome, Kita-ku**
**Osaka-shi, Osaka-fu(JP)**

(72) Inventor: **Kagiya, Tsutomu**
**3-16, Kaguraoka-cho Yoshida Sakyo-ku**
**Kyoto-shi Kyoto-fu(JP)**
Inventor: **Abe, Mitsuyuki**
**6-3, Kaguraoka-cho Yoshida Sakyo-ku**
**Kyoto-shi Kyoto-fu(JP)**
Inventor: **Nishimoto, Seiichi**
**1-17-20, Suzaku**
**Nara-shi Nara-ken(JP)**
Inventor: **Shibamoto, Yuta**
**No. 609, Neocopo Rakuhoku 13-2,**
**Kamiokubo-cho**
**Tanaka Sakyo-ku Kyoto-shi Kyoto-fu(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**CHEMICAL ABSTRACTS, vol. 105, n0. 18, 3rd November 1986, pages 489-490, abstract no. 160447r**

**CHEMICAL ABSTRACTS, vol. 107, no. 9, 31st August 1987, page 323, abstract no. 73465t**

**CHEMICAL ABSTRACTS, vol. 105, no. 15, 13th October 1986, page 342, abstract no. 130194p**

Inventor: **Shimokawa, Kazuhiro**
**2-18-2, Shinzaike**
**Settsu-shi Osaka-fu(JP)**
Inventor: **Hisanaga, Yorisato**
**1-1-19, Yamatedai**
**Ibaraki-shi Osaka-fu(JP)**
Inventor: **Nakada, Tatsuo**
**3-16-6, Hoshin Higashiyodogawa-ku**
**Osaka-shi, Osaka-fu(JP)**
Inventor: **Yoshizawa, Toru**
**1-7-26, Misaki Suminoe-ku**
**Osaka-shi Osaka-fu(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

## Description

### BACKGROUND OF THE INVENTION

### Field of the Invention

The present invention relates to novel fluorine-containing nitroimidazole derivatives and a radiosensitizer comprising the same, more particularly, a radiosensitizer comprising a specific fluorine-containing nitroimidazole derivatives such as 2-nitroimidazole compound having a fluorine containing group at the 1-position, which facilitates inactivation of intractable hypoxic cells in malignant tumors by irradiation.

### DESCRIPTION OF THE RELATED ART

To suppress reproduction or growth of malignant tumor cells, radiation exposure and administration of antitumor compounds or immunity substances are known and actually employed independently or in combination with surgical therapy. Among them, the radiation exposure has been employed for a long time.

A hypoxic cell sensitizer (or radiosensitizer) which is a drug for increasing sensitivity of the hypoxic cells against the radiation has been developed since it is promising means for increasing effects of radiotherapy.

Hitherto, various hypoxic cell sensitizers have been developed (cf. "Gan to Kagakuryoho" (Cancers and chemotherapy), Vol. 8, No. 11, November 1981, 1659).

1-(2-Nitro-1-imidazolyl)-3-methoxy-2-propanol (Misonidazole), which is one of typical hypoxic cell sensitizers, is about twice as effective as when no Misonidazole is used. However, it is hardly administered in an effective amount since it has strong neurotoxicity. No sensitizing effect was confirmed from the results obtained by administering it in human beings (cf. Adams G.E. et al, Int. J. Radiat. Biol., 19, 575-585, 1971 cited in the above "Gan to Kagakuryoho").

To increase sensitizing activity of the radiation and simultaneously to decrease the neurotoxicity, nitrotriazole derivatives and nitroimidazole derivatives have been studied (cf. Japanese Patent Kokai Publication Nos. 194019/1986 and 12763/1987). However, the conventional derivatives have insufficient radiosensitization.

It has been found that the radiosensitizing function of the azole compounds is attributed to their azole rings while the side chain contributes to their solubility in oils and pharmacological characteristics (Int. J. Radiat. Biol., 35, 1979, 151).

Compounds having a fluorine atom at a specific position in a molecule have been increasingly used as medicines because of mimic effects of the fluorine atom or modification of methabolic inhibition effect and solbility in oils (cf. "Kagakuno Ryoiki" (Chemical Fields), 35, 441 (1981)).

### SUMMARY OF THE INVENTION

One object of the present invention is to provide a novel fluorine-containing nitroimidazole derivative having a partially or completely fluorinated substituent on its imidazole ring.

Another object of the present invention is to provide a fluorine-containing radiosensitizer which increases sensitivity of the hypoxic cells against radiation but has improved pharmacological characteristics and low toxicity and neurotoxicity.

Accordingly, the present invention provides a 2-nitroimidazole derivative of the formula:

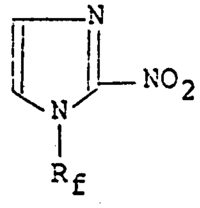

(i)

wherein $R_f$ is a fluorine-containing organic group which has at least one fluorine atom on a carbon atom which bonds to the nitrogen atom of the imidazole ring or a carbon atom which bonds to said carbon atom and a radiosensitizer comprising said nitroimidazole derivative.

BRIEF DESCRIPTION OF THE DRAWINGS

Fig. 1 is a graph showing the relationship between ER in vivo and doses of the compounds (3) and (7) and the comparative compound (c), and

Fig. 2 is graphs showing the relationships between the concentration of the administered compounds in plasm and tissue and the time after intravenous administration.

DETAILED DESCRIPTION OF THE INVENTION

In the nitroimidazole derivative (i) of the present invention, $R_f$ is a group of the formula:

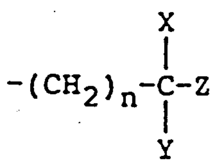

(ii)

wherein:

n is 0 or 1;

X is a hydrogen atom or a fluorine atom;

Y is a fluorine atom, a chlorine atom, a trifluoromethyl group, a methyl group or a hydroxyl group, or X and Y together represent =O;

Z is a hydrogen atom, a fluorine atom, a $C_1$-$C_5$ alkyl or fluoroalkyl group which may be substituted with hydroxyl, or Z is a group represented by one of the formulae (iii)-( x):

$-(CHE)_m-CO-OR_1$ (iii)

wherein $R_1$ is a hydrogen atom or a $C_1$-$C_5$ alkyl or fluoroalkyl group, E is a hydrogen atom or a fluorine atom and m is 0 or 1;

$-CO-R_2$ (iv)

wherein $R_2$ is a $C_1$-$C_5$ alkyl or fluoroalkyl group;

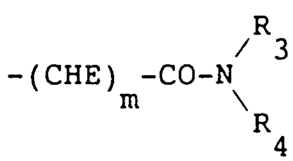

(v)

wherein $R_3$ and $R_4$ are the same or different and a hydrogen atom, a hydroxyl group or a $C_1$-$C_5$ alkyl or fluoroalkyl group which may be substituted with a hydroxyl group or a $C_1$-$C_5$ alkoxy group or an amide group, or $R_3$ and $R_4$ form a 3 to 6 membered ring together with the nitrogen atom to which they bond, and E and m are the same as defined above;

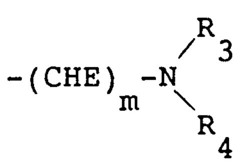

(vi)

wherein $R_3$, $R_4$, E and m are the same as defined above;

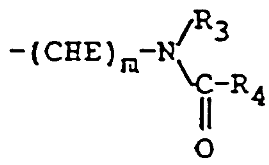

(vii)

wherein $R_3$, $R_4$, E and m are the same as defined above;

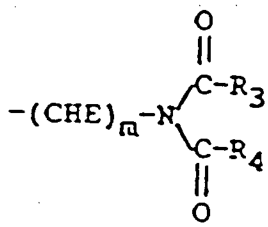

(viii)

wherein $R_3$, $R_4$, E and m are the same as defined above;

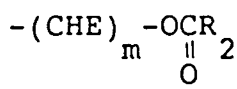

(ix)

wherein $R_2$, E and m are the same as defined above;

$-(CHE)_m-A-R_5$ (x)

wherein E and m are the same as defined above, A is an oxygen atom or a sulfur atom, and $R_5$ is a hydrogen atom, a group represented by either formula (xi) or formula (xii) defined below, or a $C_1$-$C_5$ alkyl or a fluoroalkyl group which may be substituted with a hydroxyl group, a $C_1$-$C_5$ alkoxyl group or a $C_1$-$C_5$ oxyacyl group;

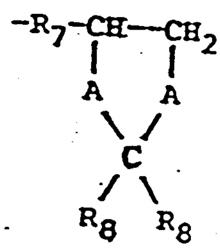

(xi)

wherein $R_7$ is a $C_1$-$C_7$ alkylene group, $R_8$ is a $C_1$ -$C_3$ alkyl group and A is the same as defined above;

$-CO-R_6$ (xii)

wherein $R_6$ is a $C_1$-$C_5$ alkyl group;
Y and Z may together form $=CF-CF_3$ or $=CHOR_6$ in which $R_6$ is the same as defined above.

At least one of the substituents X, Y, Z is a fluorine atom or a fluorine containing group. However, $R_f$ is not $-CH_2F$, $-CHF_2$, $-CH_2CH_2F$ or $-CH_2C(OH)HCH_2F$. The 2-nitroimidazole derivatives having such groups as $R_f$ have higher acute toxicity than misonidazole. Further, $R_f$ is not $CH_2CH(OH)CH_2OCH_2CF_3$ since the 2-nitroimidazole derivative with such group is not superior to misonidazole (cf. A. M. Rauth et al, Chem. Abs., Vol. 105, No. 23, 04.12.78, page 46, abstract no. 191005a).

Preferred examples of the group $R_f$ are as follows:

(1) $-CH_2CF_2COOCH_3$
(2) $-CH_2CF_2CONHCH_2CH_2OCH_3$
(3) $-CH_2CF_2CONHCH_2CH_2OH$
(4) $-CH(CF_3)-CH_2CONH(CH_2)_2OCH_3$
(5) $-CH(CF_3)-CH_2COOCH_3$
(6) $-CH_2CH(OH)CH_2OCH_2CF_2CF_2H$
(7) $-CH_2CHFCH_2OCH_3$
(8) $-CH_2CHFCH_2OCOCH_3$
(9) $-CH_2CHFCH_2OH$
(10) $-CH_2CH=CHOCH_2CF_3$
(11) $-CF=CFCF_3$
(12) $-CF_2CF_2H$
(14) $-CH_2CHFCONH(CH_2)_2OH$
(15) $-CH_2CF_3$
(16) $-CH_2CF_2COCH_3$
(17) $-CH_2COCF_3$
(18) $-CH_2COCHF_2$
(19) $-CH_2COCH_2F$

(20) 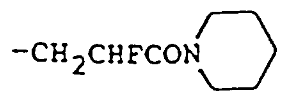

(21) 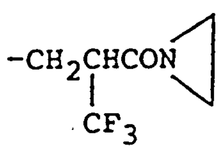

(22) 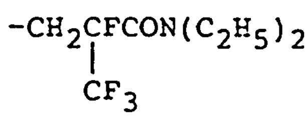

(23) 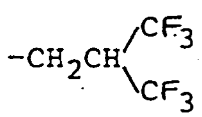

(24) 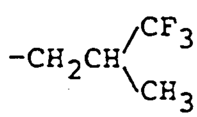

(25) $-CH_2CF_2CH_2OH$

(26) 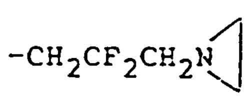

(27) $-CH_2CF_2CH_2SCH_3$
(28) $-CF_2CONH(CH_2)_2OH$

(29) 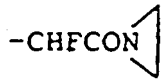

(30) 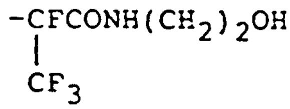

(31) 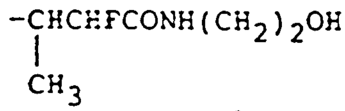

(32) 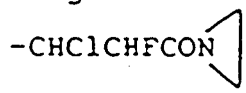

(34) $-CONH(CH_2)_3CF_3$
(35) $-CH_2CH_2CHF_2$
(37) $-CH_2CHFCOOCH_3$
(36) $-CH_2CHFCONHCH_2CH_2OCH_3$
(39) $-CH_2CF_2CH_2NH_2$
(40) $-CH_2CF_2CH_2NHCOCH_3$
(41) $-CH_2CF_2CH_2NHCOCH_2CH_2OH$

(42)

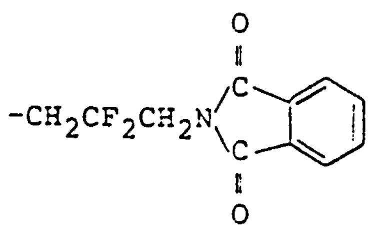

(43) $-CH_2CF_2CONH_2$
(44) $-CH_2CF_2CONHOH$
(45) $-CH_2CF_2CONHCH_2CH_2NH_3Cl$
(46) $-CH_2CF_2CH_2NHCH_2CH_2OCH_3$
(47) $-CH_2CHFCONHCH_2CH_2OH$
(48) $-CH_2CF_2CONHCH_2CH_3$
(49) $-CH_2CF_2CONHCH_2CH_2CH_3$
(50) $-CH_2CF_2CONH(CH_2)_5CH_3$
(51) $-CH_2CF_2CONHCH_2CF_3$
(52) $-CH_2CF_2CONH(CH_2)_3CF_3$
(53) $-CH_2CF_2CONHCH(CH_3)_2$
(54) $-CH_2CF_2CONHC(CH_3)_2$

(55) 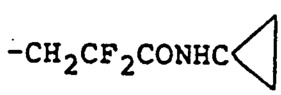

(56) $-CH_2CF_2CONH(CH_2)_2OCH_2CH_3$
(57) $-CH_2CF_2CONH(CH_2)_3OCH_2CH_3$
(58) $-CH_2CF_2CONH(CH_2)_3OCH_3$
(59) $-CH_2CF_2CONH(CH_2)_3OH$
(60) $-CH_2CF_2CONHCH_2CH(OH)CH_3$
(61) $-CH_2CF_2CONH(CH_2)_2O(CH_2)_2OH$
(62) $-CH_2CF_2CONHCH_2CH_2CONH_2$
(63) $-CH_2CHFCH_2OCH_2CH_2OCH_3$

(64) 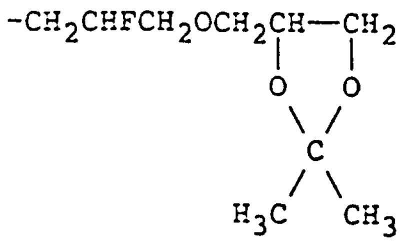

(65) $-CH_2CHFCH_2OCH_2CH(OH)CH_2OH$

(66) 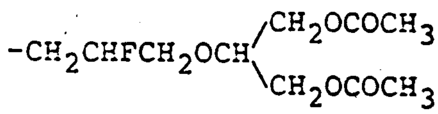

(67) 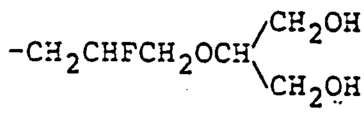

(68) $-CH_2CHFCH_2OCH_2CH_2OH$

(69) 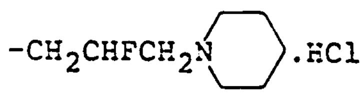

The nitroimidazole derivative (i) of the present invention may be prepared as follows:

(A) When $R_f$ is a fluoroalkyl group:

(1) A fluorine-containing epoxy compound is addition reacted with 2-nitroimidazole to form a fluoroalkyl group having a hydroxyl group as the group $R_f$.

The reaction temperature is from 0 to 100 ° C, preferably from 50 to 70 ° C. Although no solvent is required, the reaction may be carried out in a solvent such as alcohol, dioxane and the like.

(2) The hydroxyl group in the compound obtained in the above (1) is fluorinated with a suitable fluorinating agent (e.g. diethylaminosulfatrifluoride (DAST)).

The reaction is carried out in an aprotic solvent (e.g. methylene chloride, chloroform and ether) at a temperature of 0 to 50 ° C.

(3) A fluoroolefin is addition reacted with 2-nitroimidazole.

This addition reaction is carried out in an aprotic solvent (e.g. acetonitrile and dimethylformamide) in the presence of a base at a temperature of 0 to 100 ° C.

(B) When $R_f$ is a fluoroester group:

(1) A fluorine-containing $\alpha,\beta$-unsaturated carbonyl compound is addition reacted with 2-nitroimidazole.

The reaction is carried out in an aprotic solvent (e.g. dioxane and tetrahydrofuran) in the presence of an acid or a base at a temperature of 30 to 120 ° C.

(2) A fluorooxetane is addition reacted with 2-nitroimidazole.

The reaction is carried out in an alcohol (e.g. methanol and ethanol) at a temperature of 0 to 50 ° C.

(C) When $R_f$ is a fluorine-containing amide group:

The compound prepared in (B) is further reacted with an amine.

This reaction is carried out in the absence of a solvent at a temperature of 0 to 100 ° C.

(D) When $R_f$ is a fluorine-containing amine group:

(1) The compound prepared in (C) is reduced with a suitable reducing agent. Any reducing agent that does not reduce the nitro group can be used. For example, $B_2H_6$ is preferred. Any solvent that does not deactivate the reducing agent may be used. For example, tetrahydrofuan and dioxane are used.

(E) When $R_f$ is a fluorine-containing amide group:

(1) The compound prepared in (D) is reacted with carboxylic halide, carboxylic anhydride or lactone. A solvent is not necessarily required. When the carboxylic anhydride is used, a base catalyst such as pyridine or morpholine may be used.

Among the above preparation reactions, typical reactions are represented by following reaction schemes:

(A)-(1):

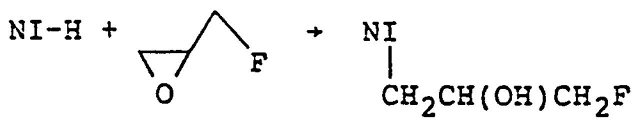

(A)-(2):

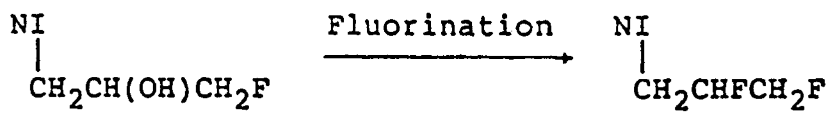


(A)-(3):

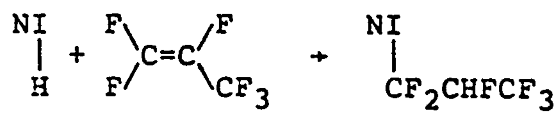

(B)-(1):

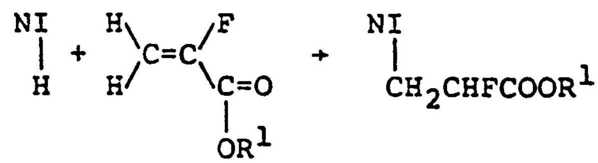

(B)-(2):

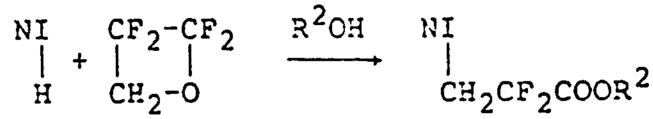


(C)-(1):

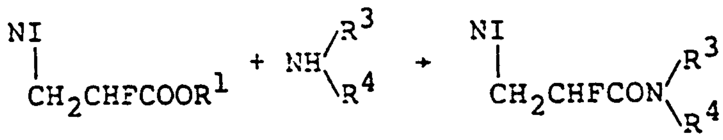

(D)-(1):

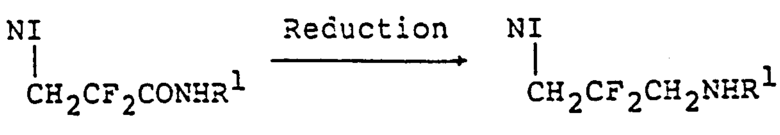


(E)-(1):

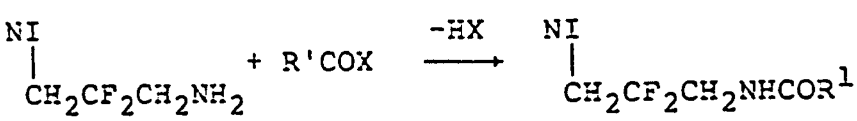


In the above formulas, $R^1$ and $R^2$ are independently a hydrogen atom or a lower alkyl group, X is a halogen atom, and "NI" means 2-nitroimidazol-1-yl.

The nitroimidazole derivative (i) of the present invention is useful as a radiosensitizer. Its dose depends on the kinds of tumor and/or the compound (i). Generally, it is from 20 to 10,000 mg in case of oral administration, from 0.5 to 10,000 mg in case of injection or 20 to 10,000 mg in case of suppository. An optimum dose may be determined by a medical practitioner according to symptom based on a kind of radiation, a radiation dose, fractionation of irradiation and the like.

The nitroimidazole compound (i) of the present invention may be administered in any suitable form. The compound (i) may be compounded with any carrier which is conventionally used in this field, and formulated by a conventional method.

The present invetion is further illustrated by the following Preparation Examples for the nitroimidazole derivatives (i) and Examples showing the radiosensitization effect of the derivatives (i).

In Examples, "NI" means 2-nitroimidazol-1-yl.

Preparation Example 1

$NI\text{-}CH_2CF_2COOCH_3$ (1)

To 1.0 g (8.8 mmol) of 2-nitroimidazole and 2.0 g (19 mmol) of sodium carbonate, 20 ml of methanol was added. To the obtained mixture, 1.8 g (14 mmol) of tetrafluorooxetane was dropwise added. After stirring for 30 minutes at room temperature, the mixture was concentrated and partitioned between ethyl acetate and water. The ethyl acetate phase was dried on magnesium sulfate and filtered. The filtrate was concentrated and subjected to isolation and purification by silica gel column chromatography to give 1.48 g of methyl 3-(2′-nitroimidazol-1′-yl)-2,2-difluoropropionate as an oil.

$^1$H-NMR ($CDCl_3$): $\delta$ = 3.89 (3H, s, $-OCH_3$), 5.26 (2H, t, $-NCH_2$, $J_{HF}$ = 14 Hz), 7.21 (2H, s, $H_{4'}$, $H_{5'}$,).

$^{19}$F-NMR (standard: TFA): 30.9.

Preparation Example 2

$NI\text{-}CH_2CF_2CONHCH_2CH_2OCH_3$ (2)

To a solution of 1.74 g (7.40 mmol) of methyl 3-(2′-nitroimidazol-1′-yl)-2,2-difluoropronionate in 5.0 ml of dioxane, 2.0 ml (23 mmol) of 2-methoxyethylamine was dropwise added and stirred at room temperature for 1 hour.

After reaction, the solution was concentrated and subjected to isolation and purification by silica gel column chromatography to give 1.75 g of 3-(2′-nitroimidazol-1′-yl)-2,2-difluoropropionic acid methoxyethylamide as an oil.

$^1$H-NMR ($CDCl_3$): δ = 3.36 (3H, s, $-CH_3$), 3.45 - 3.58 (4H, m, $-CH_2-CH_2-O-$), 5.22 (2H, t, $H_{1'}$, $J_{HF}$ = 14 Hz), 6.94 (1H, bs, -CONH-), 7.18 - 7.27 (2H, m, $H_4$, $H_5$).

$^{19}$F-NMR ($CDCl_3$; standard: TFA): 32.0.

Preparation Example 3

NI-$CH_2CF_2CONHCH_2CH_2OH$ (3)

To a solution of 1.49 g (6.34 mmol) of methyl 3-(2′-nitroimidazol-1′-yl)-2,2-difluoropronionate in 4.0 ml of methanol, 2.0 ml (33 mmol) of ethanolamine was added. After the same treatment as Preparation Example 42, 670 mg of 3-(2′-nitroimidazol-1′-yl)-2,2-difluoropronionic acid hydroxyethylamide was obtained as an oil.

$^1$H-NMR ($CDCl_3$): δ = 3.39 - 3.62 (2H, m, $-NHCH_2-$), 3.67 - 3.88 (2H, m, $-CH_2-O$), 5.22 (2H, t, $H_3$, $J_{HF}$ = 14 Hz), 6.94 (1H, bs, -CONH-), 7.21 (2H, s, $H_{4'}$, $H_{5'}$).

$^{19}$F-NMR ($CDCl_3$; standard: TFA): 31.9. m.p. 108.0 - 110.0 °C.

Preparation Example 4

NI-$CH_2CHFCH_2OCH_3$ (7)

To a solution of 914 mg (4.54 mmol) of 1-(2'-hydroxy-3'-methoxypropyl)-2-nitroimidazole in 5.0 ml of dry dioxane, 1.00 g (6.20 mmol) of DAST was dropwise added with cooling by ice and then reacted for 2 hours at room temperature with stirring. After reaction, to the solution, 5.0 ml of ethanol was added to decompose excess DAST. The solution was then concentrated and subjected to isolation and purification by silica gel column chromatography to give 480 mg of 1-(2-'-fluoro-3'-methoxypropyl)-2-nitroimidazole.

m.p. 39.0-44.0 °C.

$^1$H-NMR ($CDCl_3$): δ = 3.44 (3H, s, $-OCH_3$), 3.58 (1H, dd, $H_{3'a}$, $J_{H_{3'a}-H_{2'}}$ = 3Hz, $J_{H_{3'a}-F}$ = 3Hz), 3.80 (1H, d, $H_{3'b}$, $J_{H_{3'b}-F}$ = 3 Hz), 4.32 - 4.56 (1H, m, $H_{1'a}$), 4.65 - 4.72 (1H, m, $H_{1'b}$).

$^{19}$F-NMR ($CDCl_3$; standard: TFA): 113.6.

Preparation-Example 5

NI-$CH_2CHFCH_2OCOCH_3$ (8)

To a soluiton of 3.93 g (17.1 mmol) of 1-(2′-hydroxy-3′-acetoxypropyl)-2-nitroimidazole in 30 ml of dry dioxane, 4.00 g of DAST was dropwise added with stirring and cooling by ice. After addition, the mixture was reacted for 5 hours at room temperature with stirring. Thereafter, to the solution, 10 ml of ethanol was added to decompose excess DAST. The solution was then concentrated and partitioned between chloroform and an aqueous solution of sodium hydrogen carbonate. The chloroform phase was dried on magnesium sulfate and filtered. The filtrate was concentrated and subjected to isolation and purification by silica gel column chromatography to give 1.15 g of 1-(2′-fluoro-3′-acetoxypropyl)-2-nitroimidazole.

$^1$H-NMR ($CDCl_3$): $\delta$ = 2.15 (3H, s, $-COCH_3$), 4.12 - 5.18 (4H, m, $H_{1'}$, $H_{3'}$), 5.05 (1H, dm, $H_{2'}$, $J_{H_{2'}-F}$ = 48.0 Hz), 7.19 - 7.26 (2H, m, $H_4$, $H_5$).

$^{19}$F-NMR ($CDCl_3$; standard: TFA): 113.6.

Preparation Example 6

NI-$CH_2CHFCH_2OH$ (9)

To 910 mg (3.94 mmol) of 1-(2′-fluoro-3′-acetoxypropyl)-2-nitroimidazole, 6N-HCl was added and stirred overnight at room temperature. The reaction mixture was concentrate and subjected to isolation and purification by silica gel column chromatography to give 280 mg of 1-(2′-fluoro-3′-hydroxypropyl)-2-nitroimidazole.

m.p. 84.0-89.0 °C.

$^1$H-NMR $CDCl_3$ + DMSO-$d_6$): $\delta$ = 3.48-3.97 (2H, m $H_{3'}$), 4.52-5.24 (3H, m, $H_{1'}$, $H_{2'}$), 7.19 (1H, s, $H_5$), 7.42 (1H, s, $H_4$).

$^{19}$F-NMR ($CDCl_3$ + DMSO-$d_6$; standard: TFA): 114.5.

Preparation Example 7

NI-$CH_2CF_2CH_2OH$ (25)

To a solution of 4.00 g (15.1 mmol) of 3-(2′-nitroimidazol-1′-yl)-2,2-difluoropropionic acid in 50 ml of dry THF (tetrahydrofuran) cooled to -40 °C, 1.20 g (30.4 mmol) of $NaBH_4$ was gradually added in a nitrogen stream and then 7.6 ml (60 mmol) of $BF_3O(C_2H_5)_2$ was gradually added. After addition, the reaction mixture was stirred for one hour while cooling by ice, neutralized with dilute hydrochloric acid and extracted with ethyl acetate. The extract was dried on magnesium sulfate and filtered. The filtrate was concentrated and subjected to isolation and purification by silica gel column chromatography to give 510 mg of 1-(2′,2′-difluoro-3′-hydroxypropyl)-2-nitroimidazole.

$^1$H-NMR ($CDCl_3$): $\delta$ = 3.88 (2H, t, $H_{3'}$, $J_{H_{3'}-F}$ = 12 Hz), 5.08 (1H, t, $H_{1'}$, $J_{H_{1'}-F}$ = 12 Hz), 7.22 - 7.30 (2H, m, $H_4$, $H_5$).

$^{19}$F-NMR ($CDCl_3$; standard: TFA): 33.5.

Preparation Example 8

NI-$CH_2CF_2CONHOH$ (44)

To a solution of 3.0 g (12.8 mmol) of methyl 3-(2′-nitroimidazol-1′-yl)-2,2-difluoropropionate in 30 ml of methanol, 1.0 (18.7 mmol) of hydroxyamine.hydrochloride was suspended. To the suspension, a solution of 1.0 g of potassium hydroxide in 20 ml of methanol was gradually dropwise added followed by reaction for 3 hours at room temperature. The precipitate was filtrated off. The filtrate was concentrated and subjected to isolation and purification by silica gel column chromatography to give 160 mg of 3-(2′-nitroimidazol-1′-yl)-2,2-difluoropropionylhydroxyamide.

$^1H$-NMR ($CD_3OD$): δ = 5.32 (2H, t, $H_3$, $J_{H_3-F}$ = 15 Hz), 7.22 (1H, m, $H_5$), 7.52 (1H, s, $H_4$).

$^{19}F$-NMR ($CD_3OD$; standard; TFA): 34.5.

Preparation Example 9

NI-$CH_2CF_2CONHCH_2CH(OH)CH_3$ (60)

To a solution of 1.0 g of methyl 3-(2′-nitroimidazol-1′-yl)-2,2-difluoropronipnate in 30 ml of dioxane, 1,0 g of 2-hydroxypropylamine was added and reacted at room temperature for 3 hours. Then, the reaction mixture was concentrated and subjected to isolation and purification by silica gel chromatography to give 530 mg of 3-(2′-nitroimidazol-1′-yl)-2,2-difluoropropionyl-(2″-hydroxypropyl)amide.

$^1H$-NMR (DMSO-$d_6$; standard: TMS): δ = 1.09 3H, d, $-CH_3$, $J_{H_{2''}-H_{3''}}$ = 5Hz), 3.16 (2H, t, $-NCH_3$, $J_{H_{1''}-H_{2''}}$ = $J_{H_{1''}-NH}$ = 6 Hz), 3.80 (1H, m, CH-O-), 4.88 (1H, d, -OH, $J_{H_{2''}-OH}$ = 5 Hz), 5.36 (2H, t, $CH_2CF_2$, $J_{H_1-F}$ = 15 Hz), 7.38 (1H, d, $H_{5'}$, J = 1 Hz), 7.78 (1H, s, $H_{4'}$), 9.04 (1H, t, NH, $J_{HNH-H_{1''}}$ = 4 Hz).

$^{19}F$-NMR (DMSO-$d_6$; standard: TFA): 31.0.

Preparation Example 10

NI-$CH_2CHFCH_2$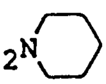 .HCl (69)

To a solution of 2.2 g (8.65 mmol) of 1-(2'-hydroxy-3'-piperidylpropyl)-2-nitroimidazole in 20 ml of dry dioxane, 2.5 g (15.5 mmol) of DAST was dropwise added with cooling by ice and reacted for 5 hours at room temperature. After reaction, to the solution, 10 ml of methanol was added to decompose excess DAST. Then, the solution was concentrated and partitioned between chloroform and water. The pH of the aqueous phase was adjusted to 12 by the addition of an aqueous solution of sodium hydroxide and the liquid was again partitioned between chloroform and water. The chloroform phase was dried on magnesium sulfate and filtered. The filtrate was concentrated and subjected to isolation and purification by silica gel chromatography to give 1-(2'-fluoro-3'-piperidylpropyl)-2-nitroimidazole, which was treated with hydrochloric acid to form a hydrochloride and crystallized from ethanol to give 560 mg of crystalline hydrochloride of 1-(2'-fluoro-3'-piperidylpropyl)-2-nitroimidazole.

$^1H$-NMR (DMSO-$d_6$): $\delta$ = 1.22 - 2.18 (6H, m, piperidyl), 2.88 - 3.65 (4H, m, piperidyl), 3.50 - 3.98 (2H, m, $H_{3'}$), 4.72 - 4.88 (1H, m, $H_{1'a}$), 5.00 - 5.13 (1H, m, $H_{1'b}$), 5.76 (1H, dm, $H_{2'}$, $J_{H_{2'}-F}$ = 51 Hz), 7.38 (1H, d, $H_5$, $J_{H_5-H_4}$ = 1.0 Hz), 7.86 (1H, d, $H_4$, $J_{H_4-H_5}$ = 1.0 Hz).

$^{19}F$-NMR (DMSO-$d_6$; standard: TFA): 106.1.

Example 1

Radiosensitization effect on cells (ER in vitro)

To examine the in vitro radiosensitization effect of the present nitroimidazole derivative (i), 100,000 cells of Chinese hamster V-79 cells were cultured in monolayer in a culture dish, and the V-79 cells in a log phase were prepared.

A solution of a predetermined concentration of a compound to be examined in a medium as added to the dish. After standing for 60 minutes at 37 °C, the dish was placed in a closed vessel at room temperature. Then, the vessel was purged with nitrogen for 10 minutes to exclude oxygen and X-ray was irradiated at a dose rate of 1.6 Gy/min.

After the irradiation, the cells were washed with phosphate buffer and digested with trypsin into single cells. Then, a predetermined amount of the cells was introduced into 5 ml of a culture medium in a culture dish and cultured for 7 days at 37 °C. After staining and washing with water, the number of colonies formed was counted.

The results are shown in following Table 1 as ER in vitro.

Examples 2

Radiosensitization effect on tumor transplanted in animal (ER in vivo)

To both thighs of male Balb/c mouse (8 weeks; 4 mice in a group), $10^5$ of EMT-6 tumor cells were subcutaneously inoculated. After the diameter of the tumor reached abut 1 cm, a solution of a compound to be examined in saline at a predetermined concentration was intraperitonealy administered. After the time at which the maximum concentration in the tumor (shown in Table 1) was reached, X-ray was irradiated at 4.5 Gy/min. for 5 minutes and then the mouse was sacrificed.

After the mouse was systemically sterilized with 70 % ethanol, the tumor was taken out and the tissue was homogenized. Then, 22 ml of trypsin was added to the homogenate and stirred for 50 minutes at 37 °C. The number of cells in the supernatant was counted. A predetermined amount of the cells was introduced in a plastic plate having a diameter of about 5 cm. To the plate, 5 ml of medium was added and cultured in a $CO_2$ incubator. The plates containing the irradiated and unirradiated cells were removed from the incubator after 9 days and 10 days incubation, respectively. The cells were fixed by methanol and stained by Giemsa stain. Then, the number of colonies formed were counted.

The survival rate was calculated with using the unirradiated cells as control. The results are shown as ER in vivo in following Table 1. In addition, the relationship between the administered amount of the compounds (3) or (7) or the comparative compound (c) (Misonidazole) and the ER in vivo is shown in Fig. 1.

Table 1

| Compound | $R_f$ | ER in vitro (mM) | ER in vivo (mg/kg) [min.]*2) | $LD_{50}$ *1) (g/kg) |
|---|---|---|---|---|
| (1) | $-CH_2CF_2COOCH_3$ | 1.40 (1.0) | --- | --- |
| (2) | $-CH_2CF_2CONH(CH_2)_2OCH_3$ | 1.61 (1.0) | 1.26 (100) | --- |
| (3) | $-CH_2CF_2CONH(CH_2)_2OH$ | 1.58 (1.0) | 1.48 (100) [20] | 2.3 |
| (7) | $-CH_2CHFCH_2OCH_3$ | 1.62 (1.0) | 1.60 (100) | 0.4 |
| (9) | $--CH_2CHFCH_2OH$ | 1.58 (1.0) | 1.58 (100) | --- |
| (25) | $-CH_2CF_2CH_2OH$ | 1.55 (1.0) | 1.44 (100) [30] | 0.4 |
| (44) | $-CH_2CF_2CONHOH$ | 1.46 (1.0) | 1.25 (100) | --- |
| (60) | $-CH_2CF_2CONHCH_2CH(OH)CH_3$ | 1.49 (1.0) | 1.12 (100) [20] | --- |
| (69) | $-CH_2CHFCH_2N$ 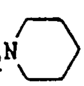 .HCl | 1.78 (1.0) | 1.45 (100) | --- |
| Comparative (a) | $-CH_2CHCl$<br>\|<br>OH | 1.78 (1.0) | 1.50 (200) | --- |
| Comparative (b) | $-CH_2CHCH_2F$<br>\|<br>OH | 1.77 (1.0) | 1.50 (200) | --- |
| Comparative (c) | $-CH_2CHCH_2OCH_3$<br>\|<br>OH<br>(Misonidazole) | --- | 1.42 (100) [20] | 1.3 |

Note: *1) For determination of $LD_{50}$, female ICR mice of 5 weeks were used.
*2) The time at which the maximum concentration in the tumor was reached. In Examples in which no time was indicated, X-ray was irradiated after 40 minutes from administration.

Example 3

Evaluation of radiosensitization effect through the Growth Delay method

To a right thigh of female C3H mouse (8 weeks, 6 to 10 mice in a group), SCC VII tumor cells were subcutaneously inoculated. After about two weeks at which the tumor reached 10 mm, a solution of a

compound to be examined in saline at a predetermined concentration was intraperitonealy administered (100 mg of the compound per kg). After 20 to 40 minutes from the administration, X-ray was irradiated at 4.5 Gy/min.

Every day, a longest radius (a) and a shortest radius (b) of the tumor were measured and the size of the tumor was calculated according to the following equation:

$$A_x = \frac{\pi \times a \times b}{4}$$

The calculated value $A_x$ was divided by the value before irradiation $A_0$ to obtain a relative volume $A_x/A_0$. The relative volume for each group were averaged and plotted against the observation period (days). From the obtained curve, the number of days at which $A_x/A_0$ is 2 ($\tau_2$, days) was calculated. From this value, a sensitization effect ratio (SER) was calculated for the case in which the sensitizer was used. The results when the compound (3) or the comparative compound (c) was used are shown in Table 2.

Table 2

| Treatment | $\tau$ (days) | SER |
|---|---|---|
| (None) | 4.5 | --- |
| Irradiation (20 Gy) | 8.0 | 1.00 |
| Irradiation (30 Gy) | 12.8 | 1.50 |
| Comparative compound (c) + Irradiation (20 Gy) | 14.7 | 1.59 |
| Compound (3) + Irradiation (20 Gy) | 17.3 | 1.87 |
| Irradiation (40 Gy) | 20.0 | 2.00 |

Example 4

Distribution of the radiosensitizer in a mouse body

To a right thigh of each of eight female C3H mice (8 weeks), SCC VII tumor cells were subcutaneously inoculated. After about two weeks at which the tumor reached 10 mm, a solution of a compound to be examined in saline at a predetermined concentration was intravenously administered (for example, 200 mg of the compound per kg). After 5, 10, 15, 20, 30, 40, 60 and 90 minutes from the adminstration, an amount of blood was sampled from the eye socket and then the mice were sacrificed. The tumor and the brain were taken out and weighed. They were homogenized in 10 time volume of pure water. Then, to the homogenate, methanol of two time volume of the homogenate was added and thoroughly stirred followed by centrifugation. The supernatant was analyzed with chromatography to find the distribution of the compound in each organ. The results for the compound (3) and the comparative compound (c) are shown in Fig. 2.

**Claims**

**Claims for the following Contacting States : AT, BE, CH, DE, FR, GB IT, LI, LU, NL, SE**

**1.** A 2-nitroimidazol derivative of the formula:

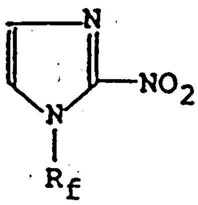

(i)

wherein $R_f$ is a group of the formula:

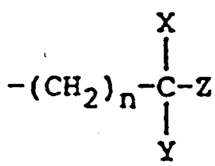

(ii)

wherein:

- n is 0 or 1;
- X is a hydrogen atom or a fluorine atom;
- Y is a fluorine atom, a chlorine atom, a trifluoromethyl group, a methyl group or a hydroxyl group, or X and Y together represent =O;
- Z is a hydrogen atom, a fluorine atom, a $C_1$-$C_5$ alkyl or fluoroalkyl group which may be substituted with hydroxyl, or Z is a group represented by one of the formulae (iii)-( x):

$-(CHE)m-CO-OR_1$ (iii)

wherein $R_1$ is a hydrogen atom or a $C_1$-$C_5$ alkyl or fluoroalkyl group, E is a hydrogen atom or a fluorine atom and m is 0 or 1;

$-CO-R_2$ (iv)

wherein $R_2$ is a $C_1$-$C_5$ alkyl or fluoroalkyl group;

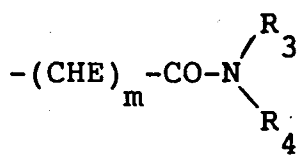

(v)

wherein $R_3$ and $R_4$ are the same or different and a hydrogen atom, a hydroxyl group or a $C_1$-$C_5$ alkyl or fluoroalkyl group which may be substituted with a hydroxyl group or a $C_1$-$C_5$ alkoxy group or an amide group, or $R_3$ and $R_4$ form a 3 to 6 membered ring together with the nitrogen atom to which they bond, and E and m are the same as defined above;

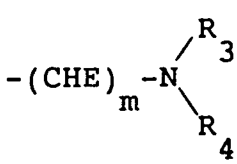

(vi)

wherein $R_3$, $R_4$, E and m are the same as defined above;

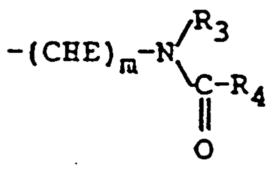

(vii)

wherein $R_3$, $R_4$, E and m are the same as defined above;

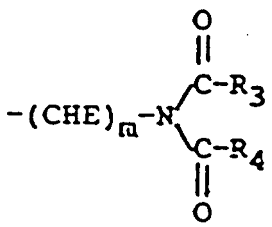

(viii)

wherein $R_3$, $R_4$, E and m are the same as defined above;

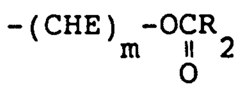

(ix)

wherein $R_2$, E and m are the same as defined above;

$-(CHE)_m-A-R_5$ ( x)

wherein E and m are the same as defined above, A is an oxygen atom or a sulfur atom, and $R_5$ is a hydrogen atom, a group represented by either formula (xi) or formula (xii) defined below, or a $C_1$-$C_5$ alkyl or a fluoroalkyl group which may be substituted with a hydroxyl group, a $C_1$-$C_5$ alkoxyl group or a $C_1$-$C_5$ oxyacyl group;

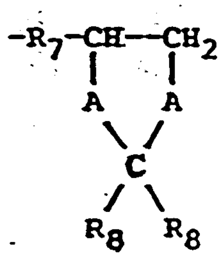

(xi)

wherein $R_7$ is a $C_1$-$C_7$ alkylene group, $R_8$ is a $C_1$-$C_3$ alkyl group and A is the same as defined above;

$-CO-R_6$ (xii)

wherein $R_6$ is a $C_1$-$C_5$ alkyl group;
Y and Z may together form $=CF-CF_3$ or $=CHOR_6$ in which $R_6$ is the same as defined above;
provided that at least one of the substituents X, Y and Z is a fluorine atom or a fluorine-containing group; when Y is a fluorine atom and n is 0 or 1, at least one of X and Z is not a hydrogen atom; when n is 0, the group -CXYZ is not a difluoromethyl group; and that when n, X and Y respectively represent 1, H and OH, then Z does not represent $-CH_2F$.

**2.** A radiosensitizer comprising, as an active component, a 2-nitroimidazol derivative as claimed in claim

1.

**Claim for the following Contracting State : ES**

**1.** A method for the manufacture of a radio-sensitizer for the treatment of intractable hypoxic cells in malignant tumors by compounding

a 2-nitroimidazol derivative of the formula:

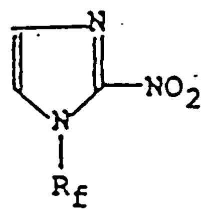

(i)

wherein $R_f$ is a group of the formula:

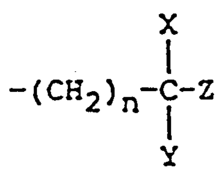

(ii)

wherein:

- n is 0 or 1;
- X is a hydrogen atom or a fluorine atom;
- Y is a fluorine atom, a chlorine atom, a trifluoromethyl group, a methyl group or a hydroxyl group, or X and Y together represent =O;
- Z is a hydrogen atom, a fluorine atom, a $C_1$-$C_5$ alkyl or fluoroalkyl group which may be substituted with hydroxyl, or Z is a group represented by one of the formulae (iii)-( x):

$-(CHE)_m-CO-OR_1$ (iii)

wherein $R_1$ is a hydrogen atom or a $C_1$-$C_5$ alkyl or fluoroalkyl group, E is a hydrogen atom or a fluorine atom and m is 0 or 1;

$-CO-R_2$ (iv)

wherein $R_2$ is a $C_1$-$C_5$ alkyl or fluoroalkyl group;

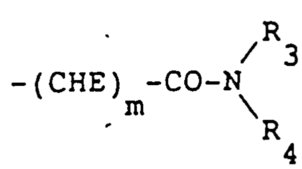

(v)

wherein $R_3$ and $R_4$ are the same or different and a hydrogen atom, a hydroxyl group or a $C_1$-$C_5$ alkyl or fluoroalkyl group which may be substituted with a hydroxyl group or a $C_1$-$C_5$ alkoxy group or an amide group, or $R_3$ and $R_4$ form a 3 to 6 membered ring together with the nitrogen atom to which they bond, and E and m are the same as defined above;

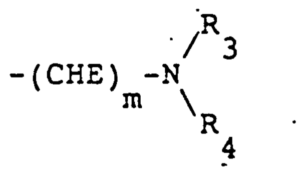

(vi)

wherein $R_3$, $R_4$, E and m are the same as defined above;

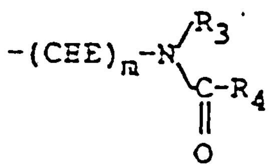

(vii)

wherein $R_3$, $R_4$, E and m are the same as defined above;

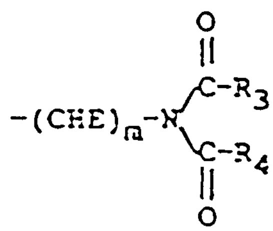

(viii)

wherein $R_3$, $R_4$, E and m are the same as defined above;

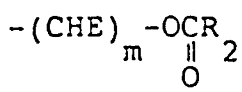

(ix)

wherein $R_2$, E and m are the sane as defined above;

$-(CHE)_m-A-R_5$ ( x)

wherein E and m are the same as defined above, A is an oxygen atom or a sulfur atom, and $R_5$ is a hydrogen atom, a group represented by either formula (xi) or formula (xii) defined below, or a $C_1$-$C_5$ alkyl or a fluoroalkyl group which may be substituted with a hydroxyl group, a $C_1$-$C_5$ alkoxyl group or a $C_1$-$C_5$ oxyacyl group;

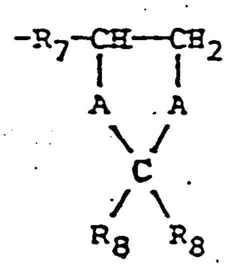

(xi)

wherein $R_7$ is a $C_1$-$C_7$ alkylene group, $R_8$ is a $C_1$-$C_3$ alkyl group and A is the same as defined above;

$-CO-R_6$ (xii)

wherein $R_6$ is a $C_1$-$C_5$ alkyl group;
Y and Z may together form $=CF-CF_3$ or $=CHOR_6$ in which $R_6$ is the same as defined above; provided that at least one of the substituents X, Y and Z is a fluorine atom or a fluorine-containing group; when Y is a fluorine atom and n is 0 or 1, at least one of X and Z is not a hydrogen atom; when n is 0, the group -CXYZ is not a difluoromethyl group; and that when n, X and Y respectively represent 1, H and OH, then Z does not represent $-CH_2F$,
with any carrier which is conventionally used in this field and formulating by a conventional method.

**Claims for the following Contracting State : GR**

1. The use of a 2-nitroimidazol derivative of the formula:

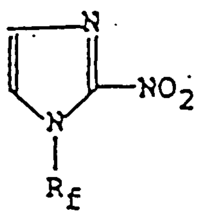

(i)

wherein $R_f$ is a group of the formula:

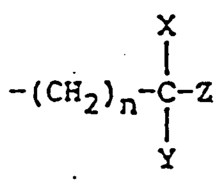

(ii)

wherein:
- n is 0 or 1;
- X is a hydrogen atom or a fluorine atom;
- Y is a fluorine atom, a chlorine atom, a trifluoromethyl group, a methyl group or a hydroxyl group, or X and Y together represent =O;
- Z is a hydrogen atom, a fluorine atom, a $C_1$-$C_5$ alkyl or fluoroalkyl group which may be substituted with hydroxyl, or Z is a group represented by one of the formulae (iii)-( x):

$-(CHE)m-CO-OR_1$ (iii)

wherein $R_1$ is a hydrogen atom or a $C_1$-$C_5$ alkyl or fluoroalkyl group, E is a hydrogen atom or a fluorine atom and m is 0 or 1;

$-CO-R_2$ (iv)

wherein $R_2$ is a $C_1$-$C_5$ alkyl or fluoroalkyl group;

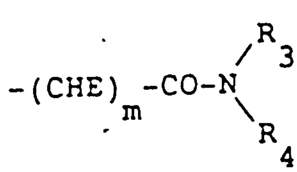

(v)

wherein $R_3$ and $R_4$ are the same or different and a hydrogen atom, a hydroxyl group or a $C_1$-$C_5$ alkyl or fluoroalkyl group which may be substituted with a hydroxyl group or a $C_1$-$C_5$ alkoxy group or an amide group, or $R_3$ and $R_4$ form a 3 to 6 membered ring together with the nitrogen atom to which they bond, and E and m are the same as defined above;

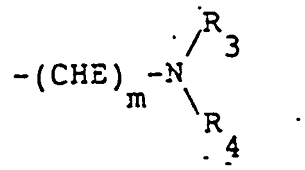

(vi)

wherein $R_3$, $R_4$, E and m are the same as defined above;

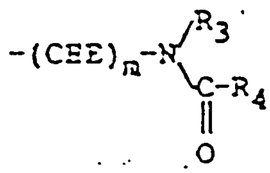

(vii)

wherein $R_3$, $R_4$, E and m are the same as defined above;

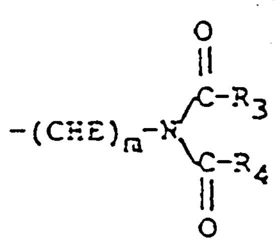

(viii)

wherein $R_3$, $R_4$, E and m are the came as defined above;

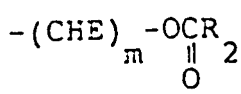

(ix)

wherein $R_2$, E and m are the sane as defined above;

$-(CHE)_m-A-R_5$ ( x)

wherein E and m are the same as defined above, A is an oxygen atom or a sulfur atom, and $R_5$ is a hydrogen atom, a group represented by either formula (xi) or formula (xii) defined below, or a $C_1$-$C_5$ alkyl or a fluoroalkyl group which may be substituted with a hydroxyl group, a $C_1$-$C_5$ alkoxyl group or a $C_1$-$C_5$ oxyacyl group;

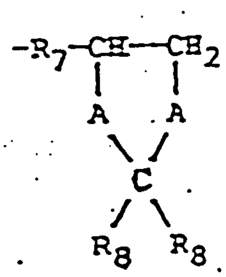

(xi)

wherein $R_7$ is a $C_1$-$C_7$ alkylene group, $R_8$ is a $C_1$-$C_3$ alkyl group and A is the same as defined above;

$-CO-R_6$ (xii)

wherein $R_6$ is a $C_1$-$C_5$ alkyl group;
Y and Z may together form $=CF-CF_3$ or $=CHOR_6$ in which $R_6$ is the same as defined above;
provided that at least one of the substituents X, Y and Z is a fluorine atone or a fluorine-containing group; when Y is a fluorine atom and n is 0 or 1, at least one of X and Z is not a hydrogen atom; when n is 0, the group -CXYZ is not a difluoromethyl group; and that when n, X and Y respectively represent 1, H and OH, then Z does not represent $-CH_2F$,

for the manufacture of a radio-sensitizer for the treatment of intractable hypoxic cells in malignant tumors.

**2.** A method for the manufacture of a radio-sensitizer for the treatment of intractable hypoxic cells in malignant tumors by compounding

a 2-nitroimidazol derivative of the formula:

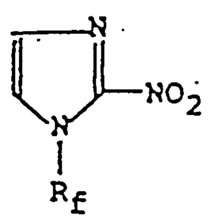

(i)

wherein $R_f$ is a group of the formula:

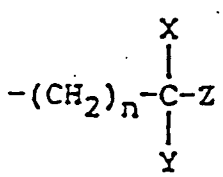

(ii)

wherein:

- n is 0 or 1;
- X is a hydrogen atom or a fluorine atom;
- Y is a fluorine atom, a chlorine atom, a trifluoromethyl group, a methyl group or a hydroxyl group, or X and Y together represent =O;
- Z is a hydrogen atom, a fluorine atom, a $C_1$-$C_5$ alkyl or fluoroalkyl group which may be substituted with hydroxyl, or Z is a group represented by one of the formulae (iii)-( x):

$-(CHE)_m-CO-OR_1$ (iii)

wherein $R_1$ is a hydrogen atom or a $C_1$-$C_5$ alkyl or fluoroalkyl group, E is a hydrogen atom or a fluorine

atom and m is 0 or 1;

-CO-$R_2$ (iv)

wherein $R_2$ is a $C_1$-$C_5$ alkyl or fluoroalkyl group;

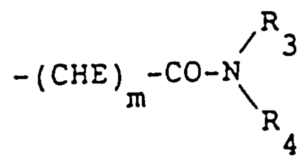

(v)

wherein $R_3$ and $R_4$ are the same or different and a hydrogen atom, a hydroxyl group or a $C_1$-$C_5$ alkyl or fluoroalkyl group which may be substituted with a hydroxyl group or a $C_1$-$C_5$ alkoxy group or an amide group, or $R_3$ and $R_4$ form a 3 to 6 membered ring together with the nitrogen atom to which they bond, and E and m are the same as defined above;

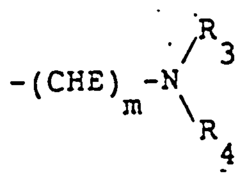

(vi)

wherein $R_3$, $R_4$, E and m are the same as defined above;

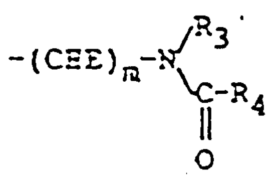

(vii)

wherein $R_3$, $R_4$, E and m are the same as defined above;

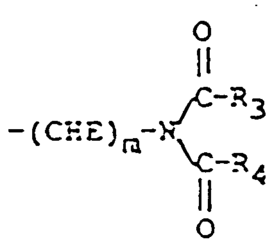

(viii)

wherein $R_3$, $R_4$, E and m are the same as defined above;

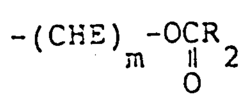

(ix)

wherein $R_2$, E and m are the same as defined above;

-$(CHE)_m$-A-$R_5$ ( x)

wherein E and m are the same as defined above, A is an oxygen atom or a sulfur atom, and $R_5$ is a hydrogen atom, a group represented by either formula (xi) or formula (xii) defined below, or a $C_1$-$C_5$ alkyl or a fluoroalkyl group which may be substituted with a hydroxyl group, a $C_1$-$C_5$ alkoxyl group or a $C_1$-$C_5$ oxyacyl group;

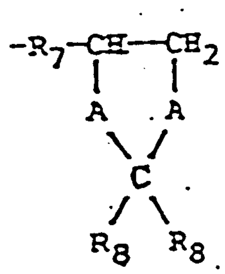

(xi)

wherein $R_7$ is a $C_1$-$C_7$ alkylene group, $R_8$ is a $C_1$-$C_3$ alkyl group and A is the same as defined above;

-CO-$R_6$ (xii)

wherein $R_6$ is a $C_1$-$C_5$ alkyl group;
Y and Z may together form =CF-$CF_3$ or =$CHOR_6$ in which $R_6$ is the same as defined above;
provided that at least one of the substituents X, Y and Z is a fluorine atom or a fluorine-containing group; when Y is a fluorine atom and n is 0 or 1, at least one of X and Z is not a hydrogen atom; when n is 0, the group -CXYZ is not a difluoromethyl group; and that when n, X and Y respectively represent 1, H and OH, then Z does not represent -$CH_2$F,
with any carrier which is conventionally used in this field and formulating by a conventional method.

**Patentansprüche**
**Patentansprüche für folgende Vertragsstaaten : AT, BE, CH, DE, FR, GB, IT, LI, LU, NL, SE**

**1.** 2-Nitroimidazolderivat der Formel:

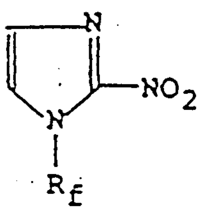

(i)

worin $R_f$ eine Gruppe der folgenden Formel ist:

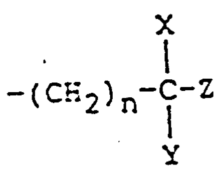

(ii)

worin:
n 0 oder 1 ist;
X ein Wasserstoffatom oder ein Fluoratom ist;
Y ein Fluoratom, ein Chloratom, eine Trifluormethylgruppe, eine Methylgruppe oder eine Hydroxylgruppe ist, oder worin X und Y zusammen =0 bilden;

Z ein Wasserstoffatom, ein Fluoratom, eine $C_1$-$C_5$ Alkyl- oder Fluoralkylgruppe ist, die mit Hydroxyl substituiert sein kann, oder worin Z eine Gruppe ist, dargestellt durch eine der Formeln (iii)-(x):

$-(CHE)_m-CO-OR_1$ (iii)

worin $R_1$ ein Wasserstoffatom oder eine $C_1$-$C_5$ Alkyl- oder Fluoralkylgruppe ist, worin E ein Wasserstoffatom oder ein Fluoratom ist und worin m 0 oder 1 ist;

$-CO-R_2$ (iv)

worin $R_2$ eine $C_1$-$C_5$ Alkyl- oder Fluoralkylgruppe ist;

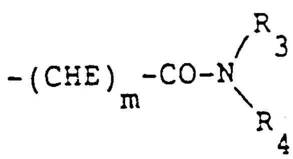

(v)

worin $R_3$ und $R_4$ gleich oder verschieden sind und ein Wasserstoffatom, eine Hydroxylgruppe oder eine $C_1$-$C_5$-Alkyl- oder Fluoralkylgruppe bedeuten, die mit einer Hydroxylgruppe oder einer $C_1$-$C_5$ Alkoxygruppe oder einer Amidgruppe substituiert sein kann, oder worin $R_3$ und $R_4$ einen drei- bis sechsgliedrigen Ring zusammen mit dem Stickstoffatom bilden, an das sie gebunden sind, und worin E und m die gleichen Bedeutungen wie oben aufweisen;

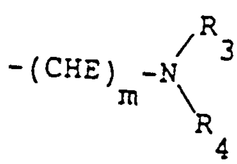

(vi)

worin $R_3$, $R_4$, E und m die gleichen Bedeutungen wie oben aufweisen;

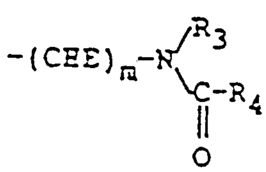

(vii)

worin $R_3$, $R_4$, E und m die gleichen Bedeutungen wie oben aufweisen;

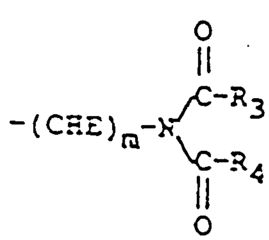

(viii)

worin $R_3$, $R_4$, E und m die gleichen Bedeutungen wie oben aufweisen;

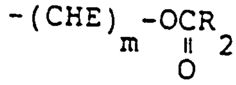 (ix)

worin $R_2$, E und m die gleichen Bedeutungen wie oben aufweisen;

$-(CHE)_m-A-R_5$ ( x)

worin E und m die gleichen Bedeutungen wie oben aufweisen, worin A ein Sauerstoffatom oder ein Schwefelatom ist, und worin $R_5$ ein Wasserstoffatom, eine Gruppe, dargestellt entweder durch die Formel (xi) oder (xii), die unten definiert sind, ist, oder eine $C_1$-$C_5$-Alkyl- oder Fluoralkylgruppe ist, die mit einer Hydroxylgruppe, einer $C_1$-$C_5$ Alkoxylgruppe oder einer $C_1$-$C_5$ Oxyacylgruppe substituiert sein kann;

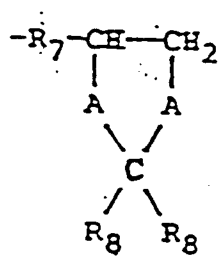 (xi)

worin $R_7$ eine $C_1$-$C_7$ Alkylengruppe ist, worin $R_8$ eine $C_1$-$C_3$ Alkylgruppe ist und worin A die gleiche Bedeutung wie oben aufweist;

$-CO-R_6$ (xii)

worin $R_6$ eine $C_1$-$C_5$ Alkylgruppe ist;
Y und z können zusammen $=CF-CF_3$ oder $=CHOR_6$ bilden, worin $R_6$ die gleiche Bedeutung wie oben angegeben aufweist;
mit dem Vorbehalt, daß zumindest eine der Substituenten X, Y und Z ein Fluoratom oder eine fluorhaltige Gruppe ist; wenn Y ein Fluoratom ist und wenn n 0 oder 1 ist, ist zumindest eines von X und Z kein Wasserstoffatom; wenn n 0 ist, ist die Gruppe -CXYZ keine Difluoromethylgruppe; und wenn n, X und Y 1, H bzw. OH bedeuten, dann ist Z nicht $-CH_2F$.

**2.** Radiosensibilisator, umfassend als einen aktiven Bestandteil ein 2-Nitroimidazolderivat nach Anspruch 1.

**Patentanspruch für folgenden Vertragsstaat : ES**

**1.** Verfahren zur Herstellung eines Radiosensibilisators zur Behandlung von hartnäckigen hypoxischen Zellen in malignen Tumoren, durch Vermischen,
eines 2-Nitroimidazolderivates der Formel:

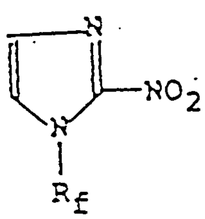 (i)

worin $R_f$ eine Gruppe der folgenden Formel ist:

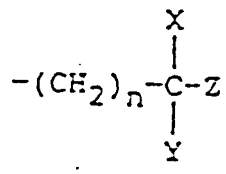

(ii)

worin:

- n 0 oder 1 ist;
- X ein Wasserstoffatom oder ein Fluoratom ist;
- Y ein Fluoratom, ein Chloratom, einr Trifluormethylgruppe, eine Methylgruppe oder eine Hydroxylgruppe ist, oder worin X und Y zusammen =0 bilden;
- Z ein Wasserstoffatom, ein Fluoratom, eine $C_1$-$C_5$ Alkyl- oder Fluoralkylgruppe ist, die mit Hydroxyl substituiert sein kann, oder worin Z eine Gruppe ist, dargestellt durch eine der Formeln (iii)-(x):

$-(CHE)_m-CO-OR_1$ (iii)

worin $R_1$ ein Wasserstoffatom oder eine $C_1$-$C_5$ Alkyl- oder Fluoralkylgruppe ist, worin E ein Wasserstoffatom oder ein Fluoratom ist und worin m 0 oder 1 ist;

$-CO-R_2$ (iv)

worin $R_2$ eine $C_1$-$C_5$ Alkyl- oder Fluoralkylgruppe ist;

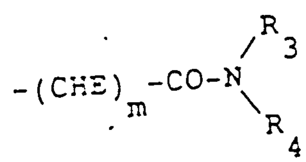

(v)

worin $R_3$ und $R_4$ gleich oder verschieden sind und ein Wasserstoffatom, eine Hydroxylgruppe oder eine $C_1$-$C_5$-Alkyl- oder Fluoralkylgruppe bedeuten, die mit einer Hydroxylgruppe oder einer $C_1$-$C_5$ Alkoxygruppe oder einer Amidgruppe substituiert sein kann, oder worin $R_3$ und $R_4$ einen drei- bis sechsgliedrigen Ring zusammen mit dem Stickstoffatom bilden, an das sie gebunden sind, und worin E und m die gleichen Bedeutungen wie oben aufweisen;

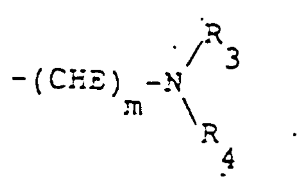

(vi)

worin $R_3$, $R_4$, E und m die gleichen Bedeutungen wie oben aufweisen;

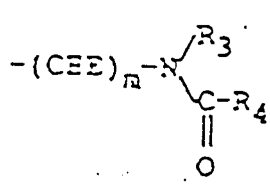

(vii)

worin $R_3$, $R_4$, E und m die gleichen Bedeutungen wie oben aufweisen;

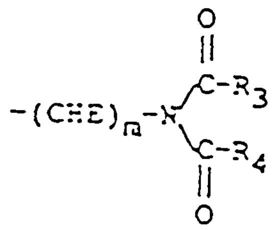

(viii)

worin $R_3$, $R_4$, E und m die gleichen Bedeutungen wie oben aufweisen;

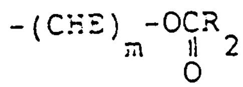

(ix)

worin $R_2$, E und m die gleichen Bedeutungen wie oben aufweisen;

$-(CHE)_m-A-R_5$ ( x)

worin E und m die gleichen Bedeutungen wie oben aufweisen, worin A ein Sauerstoffatom oder ein Schwefelatom ist, und worin $R_5$ ein Wasserstoffatom, eine Gruppe, dargestellt entweder durch die Formel (xi) oder (xii), die unten definiert sind, ist, oder eine $C_1$-$C_5$-Alkyl- oder Fluoralkylgruppe ist, die mit einer Hydroxylgruppe, einer $C_1$-$C_5$ Alkoxylgruppe oder einer $C_1$-$C_5$ Oxyacylgruppe substituiert sein kann;

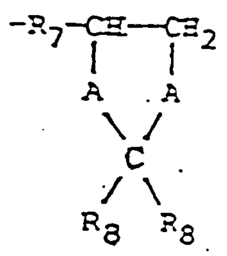

(xi)

worin $R_7$ eine $C_1$-$C_7$ Alkylengruppe worin $R_8$ eine $C_1$-$C_3$ Alkylgruppe ist und worin A die gleiche Bedeutung wie oben aufweist;

$-CO-R_6$ (xii)

worin $R_6$ eine $C_1$-$C_5$ Alkylgruppe ist;
Y und z können zusammen $=CF-CF_3$ oder $=CHOR_6$ bilden, worin $R_6$ die gleiche Bedeutung wie oben angegeben aufweist;
mit dem Vorbehalt, daß zumindest einer der Substituenten X, Y und Z ein Fluoratom oder eine fluorhaltige Gruppe ist; wenn Y ein Fluoratom ist und wenn n 0 oder 1 ist, ist zumindest eines von X und Z kein Wasserstoffatom; wenn n 0 ist, ist die Gruppe -CXYZ keine Difluoromethylgruppe; und wenn n, X und Y 1, H bzw. OH bedeuten, dann ist Z nicht $-CH_2F$,
mit irgendeinem Träger, der auf diesem Gebiet üblicherweise verwendet wird, und Formulieren durch ein übliches Verfahren.

**Patentansprüche für folgenden Vertragsstaat : GR**

1. Verwendung eines 2-Nitroimidazolderivatrd der Formel:

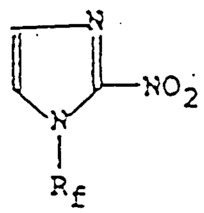 (i)

worin $R_f$ eine Gruppe der folgenden Formel ist:

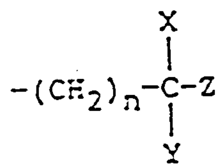 (ii)

worin:

- n 0 oder 1 ist;
- X ein Wasserstoffatom oder ein Fluoratom ist;
- Y ein Fluoratom, ein Chloratom, eine Trifluormethylgruppe, eine Methylgruppe oder eine Hydroxylgruppe ist, oder worin X und Y zusammen =0 bilden;
- Z ein Wasserstoffatom, ein Fluoratom, eine $C_1$-$C_5$ Alkyl- oder Fluoralkylgruppe ist, die mit Hydroxyl substituiert sein kann, oder worin Z eine Gruppe ist, dargestellt durch eine der Formeln (iii)-(x):

$-(CHE)_m-CO-OR_1$ (iii)

worin $R_1$ ein Wasserstoffatom oder eine $C_1$-$C_5$ Alkyl- oder Fluoralkylgruppe ist, worin E ein Wasserstoffatom oder ein Fluoratom ist und worin m 0 oder 1 ist;

$-CO-R_2$ (iv)

worin $R_2$ eine $C_1$-$C_5$ Alkyl- oder Fluoralkylgruppe ist;

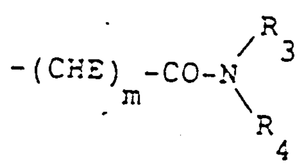 (v)

worin $R_3$ und $R_4$ gleich oder verschieden sind und ein Wasserstoffatom, eine Hydroxylgruppe oder eine $C_1$-$C_5$-Alkyl- oder Fluoralkylgruppe bedeuten, die mit einer Hydroxylgruppe oder einer $C_1$-$C_5$ Alkoxygruppe oder einer Amidgruppe substituiert sein kann, oder worin $R_3$ und $R_4$ einen drei- bis sechsgliedrigen Ring zusammen mit dem Stickstoffatom bilden, an das sie gebunden sind, und worin E und m die gleichen Bedeutungen wie oben aufweisen;

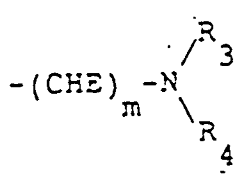 (vi)

worin $R_3$, $R_4$, E und m die gleichen Bedeutungen wie oben aufweisen;

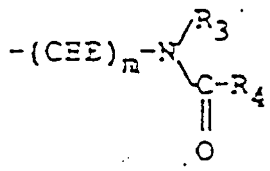

(vii)

worin $R_3$, $R_4$, E und m die gleichen Bedeutungen wie oben aufweisen;

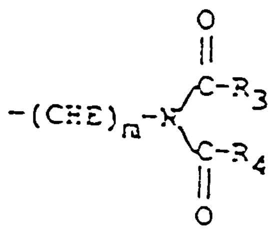

(viii)

worin $R_3$, $R_4$, E und m die gleichen Bedeutungen wie oben aufweisen;

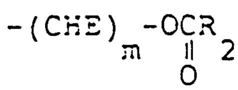

(ix)

worin $R_2$, E und m die gleichen Bedeutungen wie oben aufweisen;

$-(CHE)_m-A-R_5$ ( x)

worin E und m die gleichen Bedeutungen wie oben aufweisen, worin A ein Sauerstoffatom oder ein Schwefelatom ist, und worin $R_5$ ein Wasserstoffatom, eine Gruppe, dargestellt entweder durch die Formel (xi) oder (xii), die unten definiert sind, ist, oder eine $C_1$-$C_5$-Alkyl- oder Fluoralkylgruppe ist, die mit einer Hydroxylgruppe, einer $C_1$-$C_5$ Alkoxylgruppe oder einer $C_1$-$C_5$ Oxyacylgruppe substituiert sein kann;

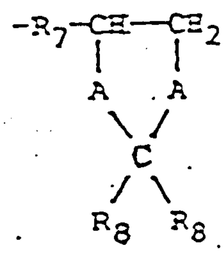

(xi)

worin $R_7$ eine $C_1$-$C_7$ Alkylengruppe ist, worin $R_8$ eine $C_1$-$C_3$ Alkylgruppe ist und worin A die gleiche Bedeutung wie oben aufweist;

$-CO-R_6$ (xii)

worin $R_6$ eine $C_1$-$C_5$ Alkylgruppe ist;
Y und z können zusammen $=CF-CF_3$ oder $=CHOR_6$ bilden, worin $R_6$ die gleiche Bedeutung wie oben angegeben aufweist;
mit dem Vorbehalt, daß zumindest eine der Substituenten X, Y und Z ein Fluoratom oder eine fluorhaltige Gruppe ist; wenn Y ein Fluoratom ist und wenn n 0 oder 1 ist, ist zumindest eines von X und Z kein Wasserstoffatom; wenn n 0 ist, ist die Gruppe -CXYZ keine Difluoromethylgruppe; und wenn

n, X und Y 1, H bzw. OH bedeuten, dann ist Z nicht $-CH_2F$,
für die Herstellung eines Radiosensibilisators für die Behandlung von hartnäckigen hypoxischen Zellen in malignen Tumoren.

**2.** Verfahren zur Herstellung eines Radiosensibilisators für die Behandlung von hartnäckigen hypoxischen Zellen in malignen Tumoren durch Vermischen eines
2-Nitroimidazolderivates der Formel:

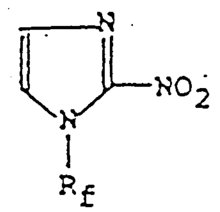

(i)

worin $R_f$ eine Gruppe der folgenden Formel ist:

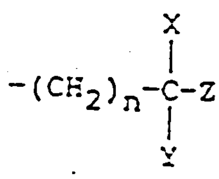

(ii)

worin:

- n 0 oder 1 ist;
- X ein Wasserstoffatom oder ein Fluoratom ist;
- Y ein Fluoratom, ein Chloratom, eine Trifluormethylgruppe, eine Methylgruppe oder eine Hydroxylgruppe ist, oder worin X und Y zusammen =0 bilden;
- Z ein Wasserstoffatom, ein Fluoratom, eine $C_1$-$C_5$ Alkyl- oder Fluoralkylgruppe ist, die mit Hydroxyl substituiert sein kann, oder worin Z eine Gruppe ist, dargestellt durch eine der Formeln (iii)-(x):

$-(CHE)_m-CO-OR_1$ (iii)

worin $R_1$ ein Wasserstoffatom oder eine $C_1$-$C_5$ Alkyl- oder Fluoralkylgruppe ist, worin E ein Wasserstoffatom oder ein Fluoratom ist und worin m 0 oder 1 ist;

$-CO-R_2$ (iv)

worin $R_2$ eine $C_1$-$C_5$ Alkyl- oder Fluoralkylgruppe ist;

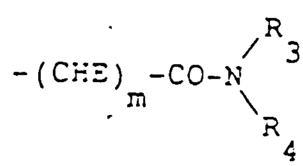

(v)

worin $R_3$ und $R_4$ gleich oder verschieden sind und ein Wasserstoffatom, eine Hydroxylgruppe oder eine $C_1$-$C_5$-Alkyl- oder Fluoralkylgruppe bedeuten, die mit einer Hydroxylgruppe oder einer $C_1$-$C_5$ Alkoxygruppe oder einer Amidgruppe substituiert sein kann, oder worin $R_3$ und $R_4$ einen drei- bis sechsgliedrigen Ring zusammen mit dem Stickstoffatom bilden, an das sie gebunden sind, und worin E und m die gleichen Bedeutungen wie oben aufweisen;

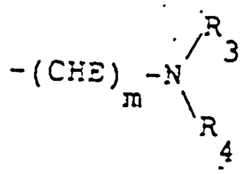

(vi)

worin $R_3$, $R_4$, E und m die gleichen Bedeutungen wie oben aufweisen;

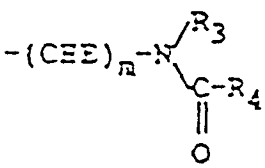

(vii)

worin $R_3$, $R_4$, E und m die gleichen Bedeutungen wie oben aufweisen;

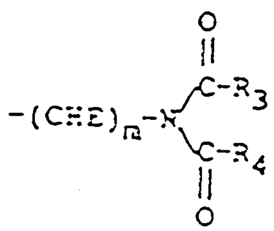

(viii)

worin $R_3$, $R_4$, E und m die gleichen Bedeutungen wie oben aufweisen;

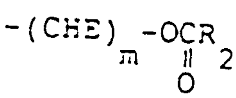

(ix)

worin $R_2$, E und m die gleichen Bedeutungen wie oben aufweisen;

$-(CHE)_m-A-R_5$ ( x)

worin E und m die gleichen Bedeutungen wie oben aufweisen, worin A ein Sauerstoffatom oder ein Schwefelatom ist, und worin $R_5$ ein Wasserstoffatom, eine Gruppe, dargestellt entweder durch die Formel (xi) oder (xii), die unten definiert sind, ist, oder eine $C_1$-$C_5$-Alkyl- oder Fluoralkylgruppe ist, die mit einer Hydroxylgruppe, einer $C_1$-$C_5$ Alkoxylgruppe oder einer $C_1$-$C_5$ Oxyacylgruppe substituiert sein kann;

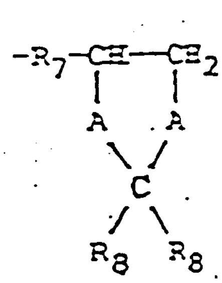

(xi)

worin $R_7$ eine $C_1$-$C_7$ Alkylengruppe ist, worin $R_8$ eine $C_1$-$C_3$ Alkylgruppe ist und worin A die gleiche Bedeutung wie oben aufweist;

$-CO-R_6$ (xii)

worin $R_6$ eine $C_1$-$C_5$ Alkylgruppe ist;
Y und z können zusammen $=CF-CF_3$ oder $=CHOR_6$ bilden, worin $R_6$ die gleiche Bedeutung wie oben angegeben aufweist;
mit dem Vorbehalt, daß zumindest eine der Substituenten X, Y und Z ein Fluoratom oder eine fluorhaltige Gruppe ist; wenn Y ein Fluoratom ist und wenn n 0 oder 1 ist, ist zumindest eines von X und Z kein Wasserstoffatom; wenn n 0 ist, ist die Gruppe -CXYZ keine Difluoromethylgruppe; und wenn n, X und Y 1, H bzw. OH bedeuten, dann ist Z nicht $-CH_2F$,
mit irgendeinem Träger, der auf diesem Gebiet üblicherweise verwendet wird und Formulieren durch ein übliches Verfahren.

**Revendications**

**Revendications pour les Etats contractants suivants : AT, BE, CH, DE, FR, GB, IT, LI, LU, NL, SE**

**1.** Dérivés de 2-nitroimidazole de formule :

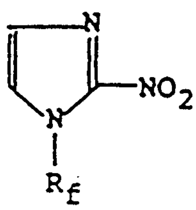

(i)

dans laquelle $R_f$ représente un groupe de formule :

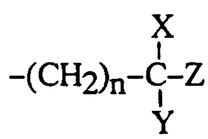

(ii)

dans laquelle :

- n vaut 0 ou 1 ;
- X représente un atome d'hydrogène ou un atome de fluor ;
- Y représente un atome de fluor, un atome de chlore, un groupe trifluorométhyle, un groupe méthyle ou un groupe hydroxyle ; ou bien X et Y, pris ensemble, représentent =0 ;
- Z représente un atome d'hydrogène, un atome de fluor, un groupe alkyle ou fluoroalkyle en $C_1$ à $C_5$, qui peut porter un groupe hydroxyle comme substituant, ou bien Z est un groupe représenté par l'une des formules (iii) à (x) :

$-(CHE)_mCO-OR_1$ (iii)

où $R_1$ représente un atome d'hydrogène ou un groupe alkyle ou fluoroalkyle en $C_1$ à $C_5$ ; E représente un atome d'hydrogène ou un atome de fluor, et m vaut 0 ou 1 ;

$-CO-R_2$ (iv)

où $R_2$ représente un groupe alkyle ou fluoroalkyle en $C_1$ à $C_5$ ;

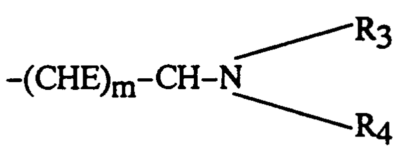

(v)

où $R_3$ et $R_4$ sont identiques ou différents et représentent chacun un atome d'hydrogène, un groupe hydroxyle ou un groupe alkyle ou fluoroalkyle en $C_1$ à $C_5$, qui peut être substitué par un groupe hydroxyle ou par un groupe alcoxy en $C_1$ à $C_5$ ou un groupe amide ; ou bien $R_3$ et $R_4$ forment, avec l'atome d'azote auquel ils sont reliés, un noyau comportant 3 à 6 chaînons ; et E et m ont le même sens que celui défini ci-dessus ;

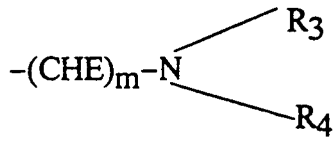

(vi)

où $R_3$, $R_4$, E et m ont le même sens que celui défini ci-dessus ;

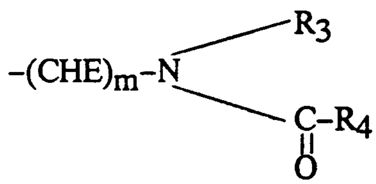

(vii)

où $R_3$, $R_4$, E et m ont le même sens que celui défini ci-dessus ;

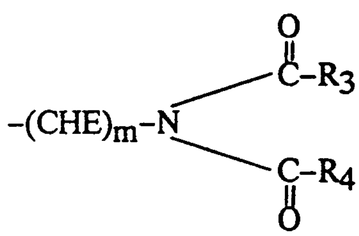

(viii)

où $R_3$, $R_4$, E et m ont le même sens que celui défini ci-dessus ;

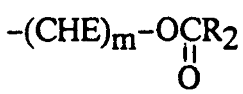

(ix)

où $R_2$, E et m ont le même sens que celui défini ci-dessus ;

$-(CHE)_m-A-R_5$ (x)

où E et m ont le même sens que celui défini ci-dessus ; A représente un atome d'oxygène ou un atome de soufre, et $R_5$ représente un atome d'hydrogène, un groupe représenté par la formule (ix) ou par la formule (xii) ci-après, ou bien un groupe alkyle ou fluoroalkyle en $C_1$ à $C_5$, qui peut être substitué par un groupe hydroxyle, un groupe alcoxyle en $C_1$ à $C_5$ ou un groupe oxyacyle en $C_1$ à $C_5$ ;

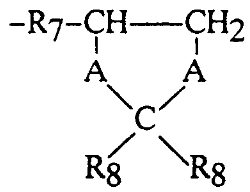

(xi)

où $R_7$ représente un groupe alkylène en $C_1$ à $C_7$, $R_8$ représente un groupe alkyle en $C_1$ à $C_3$ et A a le même sens que celui défini ci-dessus ;

$-CO-R_6$ (xii)

où $R_6$ représente un groupe alkyle en $C_1$ à $C_5$ ;
Y et Z peuvent former ensemble $=CF-CF_3$ ou $=CHOR_6$, formule dans laquelle $R_6$ a le même sens que celui défini ci-dessus ;
à la condition qu'au moins l'un des substituants X, Y et Z soit un atome de fluor ou un groupe contenant du fluor ; lorsque Y est un atome de fluor et que n vaut 0 ou 1, au moins l'un des symboles X et Z ne représente pas un atome d'hydrogène ; lorsque n est nul, le groupe -CXYZ n'est pas un groupe difluorométhyle ; et à la condition que, lorsque n vaut 1 et X et Y représentent respectivement H et OH, le symbole Z ne représentent pas $-CH_2F$

**2.** Radiosensibilisateur comprenant, à titre de constituant actif, un dérivé de 2-nitroimidazole tel que revendiqué à la revendication 1.

**Revendication pour l'Etat contractant suivant : ES**

**1.** Procédé pour la fabrication d'un radiosensibilisateur destiné à traiter des cellules hypoxiques réfractaires présentes dans des tumeurs malignes en associant ou mélangeant :
un dérivé de 2-nitroimidazole de formule :

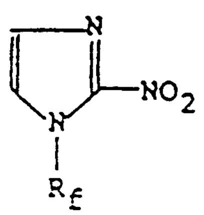

(i)

dans laquelle $R_f$ représente un groupe de formule :

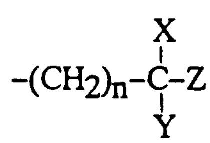

(ii)

dans laquelle :

- n vaut 0 ou 1 ;
- X représente un atome d'hydrogène ou un atome de fluor ;
- Y représente un atome de fluor, un atome de chlore, un groupe trifluorométhyle, un groupe méthyle ou un groupe hydroxyle ; ou bien X et Y, pris ensemble, représentent =O ;
- Z représente un atome d'hydrogène, un atome de fluor, un groupe alkyle ou fluoroalkyle en $C_1$ à $C_5$, qui peut porter un groupe hydroxyle comme substituant, ou bien Z est un groupe représenté par l'une des formules (iii) à (x) :

$-(CHE)_mCO-OR_1$ (iii)

où $R_1$ représente un atome d'hydrogène ou un groupe alkyle ou fluoroalkyle en $C_1$ à $C_5$ ; E représente un atome d'hydrogène ou un atome de fluor, et m vaut 0 ou 1 ;

$-CO-R_2$ (iv)

où $R_2$ représente un groupe alkyle ou fluoroalkyle en $C_1$ à $C_5$ ;

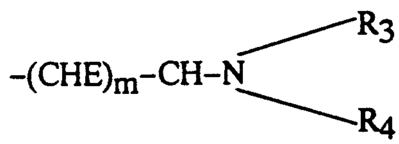

(v)

où $R_3$ et $R_4$ sont identiques ou différents et représentent chacun un atome d'hydrogène, un groupe hydroxyle ou un groupe alkyle ou fluoroalkyle en $C_1$ à $C_5$, qui peut être substitué par un groupe hydroxyle ou par un groupe alcoxy en $C_1$ à $C_5$ ou un groupe amide ; ou bien $R_3$ et $R_4$ forment, avec l'atome d'azote. auquel ils sont réliés, un noyau comportant 3 à 6 chaînons ; et E et m ont le même sens que celui défini ci-dessus ;

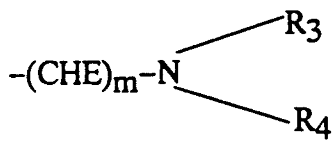

(vi)

où $R_3$, $R_4$, E et m ont le même sens que celui défini ci-dessus ;

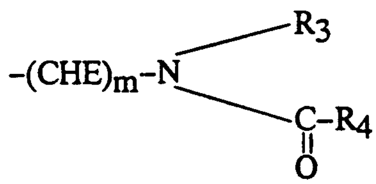

(vii)

où $R_3$, $R_4$, E et m ont le même sens que celui défini ci-dessus ;

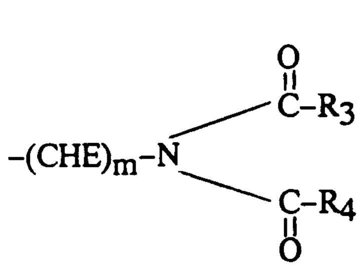

(viii)

où $R_3$, $R_4$, E et m ont le même sens que celui défini ci-dessus ;

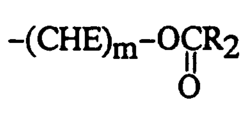

(ix)

où $R_2$, E et mont le même sens que celui défini ci-dessus ;

$-(CHE)_m-A-R_5$ (x)

où E et m ont le même sens que celui défini ci-dessus ; A représente un atome d'oxygène ou un atome de soufre, et $R_5$ représente un atome d'hydrogène, un groupe représenté par la formule (ix) ou par la formule (xii) ci-après, ou bien un groupe alkyle ou fluoroalkyle en $C_1$ à $C_5$, qui peut être substitué par un groupe hydroxyle, un groupe alcoxyle en $C_1$ à $C_5$ ou un groupe oxyacyle en $C_1$ à $C_5$ ;

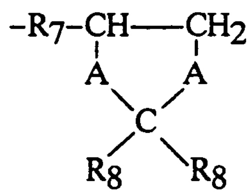

(xi)

où $R_7$ représente un groupe alkylène en $C_1$ à $C_7$, $R_8$ représente un groupe alkyle en $C_1$ à $C_3$ et A a le même sens que celui défini ci-dessus ;

$-CO-R_6$ (xii)

où $R_6$ représente un groupe alkyle en $C_1$ à $C_5$ ;

Y et Z peuvent former ensemble $=CF-CF_3$ ou $=CHOR_6$, formule dans laquelle $R_6$ a le même sens que celui défini ci-dessus ;

à la condition qu'au moins l'un des substituants X, Y et Z soit un atome de fluor ou un groupe contenant du fluor ; lorsque Y est un atome de fluor et que n vaut 0 ou 1, au moins l'un des symboles X et Z ne représente pas un atome d'hydrogène ; lorsque n est nul, le groupe -CXYZ n'est pas un groupe difluorométhyle ; et à la condition que, lorsque n vaut 1 et X et Y représentent respectivement H et OH, le symbole Z ne représentent pas $-CH_2F$,

avec n'importe quel excipient ou véhicule que l'on utilise traditionnellement dans ce domaine et en formulant par une méthode traditionnelle.

**Revendications pour l'Etat contractant suivant : GR**

**1.** Utilisation d'un dérivé de 2-nitroimidazole de formule :

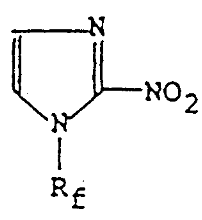

(i)

dans laquelle $R_f$ représente un groupe de formule :

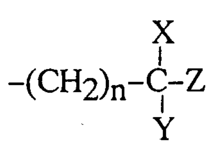

(ii)

dans laquelle :

n vaut 0 ou 1 ;

X représente un atome d'hydrogène ou un atome de fluor ;

Y représente un atome de fluor, un atome de chlore, un groupe trifluorométhyle, un groupe méthyle ou un groupe hydroxyle ; ou bien X et Y, pris ensemble, représentent =O ;

Z représente un atome d'hydrogène, un atome de fluor, un groupe alkyle ou fluoroalkyle en $C_1$ à $C_5$, qui peut porter un groupe hydroxyle comme substituant, ou bien Z est un groupe représenté par l'une des formules (iii) à (x) :

$-(CHE)_mCO\text{-}OR_1$ (iii)

où $R_1$ représente un atome d'hydrogène ou un groupe alkyle ou fluoroalkyle en $C_1$ à $C_5$ ; E représente un atome d'hydrogène ou un atome de fluor, et m vaut O ou 1 ;

$-CO\text{-}R_2$ (iv)

où $R_2$ représente un groupe alkyle ou fluoroalkyle en $C_1$ à $C_5$ ;

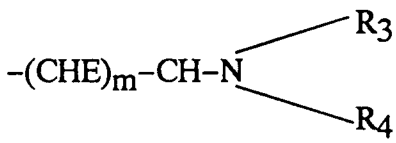

(v)

où $R_3$ et $R_4$ sont identiques ou différents et représentent chacun un atome d'hydrogène, un groupe hydroxyle ou un groupe alkyle ou fluoroalkyle en $C_1$ à $C_5$, qui peut être substitué par un groupe hydroxyle ou par un groupe alcoxy en $C_1$ à $C_5$ ou un groupe amide ; ou bien $R_3$ et $R_4$ forment, avec l'atome d'azote auquel ils sont réliés, un noyau comportant 3 à 6 chaînons ; et E et m ont le même sens que celui défini ci-dessus ;

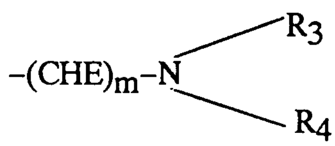

(vi)

où $R_3$, $R_4$, E et m ont le même sens que celui défini ci-dessus ;

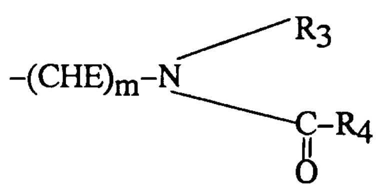

(vii)

où $R_3$, R4, E et m ont le même sens que celui défini ci-dessus ;

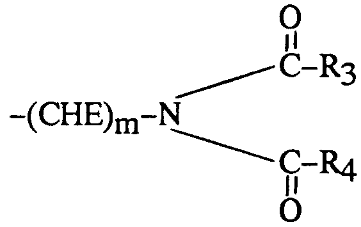

(viii)

où $R_3$, $R_4$, E et m ont le même sens que celui défini ci-dessus ;

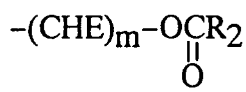

(ix)

où $R_2$, E et m ont le même sens que celui défini ci-dessus ;

$-(CHE)_m-A-R_5$ (x)

où E et m ont le même sens que celui défini ci-dessus ; A représente un atome d'oxygène ou un atome de soufre, et $R_5$ représente un atome d'hydrogène, un groupe représenté par la formule (ix) ou par la formule (xii) ci-après, ou bien un groupe alkyle ou fluoroalkyle en $C_1$ à $C_5$, qui peut être substitué par un groupe hydroxyle, un groupe alcoxyle en $C_1$ à $C_5$ ou un groupe oxyacyle en $C_1$ à $C_5$ ;

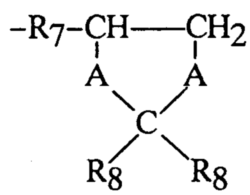

(xi)

où $R_7$ représente un groupe alkylène en $C_1$ à $C_7$, $R_8$ représente un groupe alkyle en $C_1$ à $C_3$ et A a le même sens que celui défini ci-dessus ;

$-CO-R_6$ (xii)

où $R_6$ représente un groupe alkyle en $C_1$ à $C_5$ ;
Y et Z peuvent former ensemble $=CF-CF_3$ ou $=CHOR_6$, formule dans laquelle $R_6$ a le même sens que celui défini ci-dessus ;
à la condition qu'au moins l'un des substituants X, Y et Z soit un atome de fluor ou un groupe contenant du fluor ; lorsque Y est un atome de fluor et que n vaut 0 ou 1, au moins l'un des symboles X et Z ne représente pas un atome d'hydrogène ; lorsque n est nul, le groupe -CXYZ n'est pas un groupe difluorométhyle ; et à la condition que, lorsque n vaut 1 et X et Y représentent respectivement H et OH, le symbole Z ne représentent pas $-CH_2F$,
pour la fabrication d'un radiosensibilisateur destiné à traiter des cellules hypoxiques réfractaires présentes dans des tumeurs malignes.

**2.** Procédé de fabrication d'un dérivé de 2-nitroimidazole de formule :

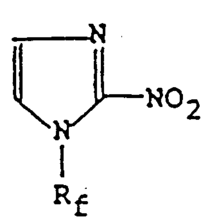

(i)

dans laquelle $R_f$ représente un groupe de formule :

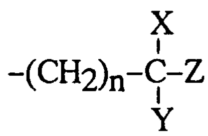

(ii)

dans laquelle :

- n vaut 0 ou 1 ;
- X représente un atome d'hydrogène ou un atome de fluor ;
- Y représente un atome de fluor, un atome de chlore, un groupe trifluorométhyle, un groupe méthyle ou un groupe hydroxyle ; ou bien X et Y, pris ensemble, représentent =O ;
- Z représente un atome d'hydrogène, un atome de fluor, un groupe alkyle ou fluoroalkyle en $C_1$ à $C_5$, qui peut porter un groupe hydroxyle comme substituant, ou bien Z est un groupe représenté par l'une des formules (iii) à (x) :

$-(CHE)_mCO-OR_1$ (iii)

où $R_1$ représente un atome d'hydrogène ou un groupe alkyle ou fluoroalkyle en $C_1$ à $C_5$ ; E représente un atome d'hydrogène ou un atome de fluor, et m vaut 0 ou 1 ;

$-CO-R_2$ (iv)

où $R_2$ représente un groupe alkyle ou fluoroalkyle en $C_1$ à $C_5$ ;

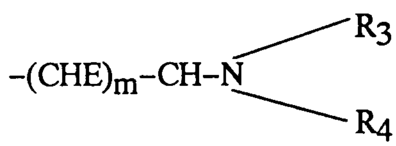

(v)

où $R_3$ et $R_4$ sont identiques ou différents et représentent chacun un atome d'hydrogène, un groupe hydroxyle ou un groupe alkyle ou fluoroalkyle en $C_1$ à $C_5$, qui peut être substitué par un groupe hydroxyle ou par un groupe alcoxy en $C_1$ à $C_5$ ou un groupe amide ; ou bien $R_3$ et $R_4$ forment, avec l'atome d'azote auquel ils sont reliés, un noyau comportant 3 à 6 chaînons ; et E et m ont le même sens que celui défini ci-dessus ;

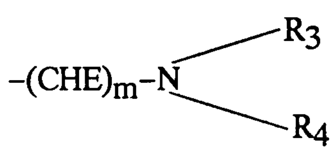

(vi)

où $R_3$, $R_4$, E et m ont le même sens que celui défini ci-dessus ;

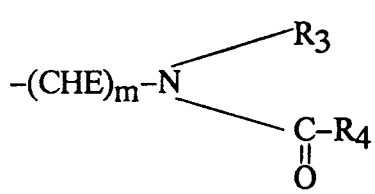

(vii)

où $R_3$, $R_4$, E et m ont le même sens que celui défini ci-dessus ;

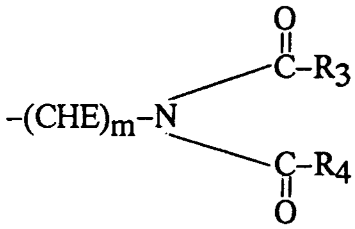

(viii)

où $R_3$, $R_4$, E et m ont le même sens que celui défini ci-dessus ;

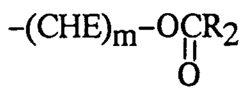

(ix)

où $R_2$, E et m ont le même sens que celui défini ci-dessus ;

$-(CHE)_m-A-R_5$ (x)

où E et m ont le même sens que celui défini ci-dessus ; A représente un atome d'oxygène ou un atome de soufre, et $R_5$ représente un atome d'hydrogène, un groupe représenté par la formule (ix) ou par la formule (xii) ci-après, ou bien un groupe alkyle ou fluoroalkyle en $C_1$ à $C_5$, qui peut être substitué par un groupe hydroxyle, un groupe alcoxyle en $C_1$ à $C_5$ ou un groupe oxyacyle en $C_1$ à $C_5$ ;

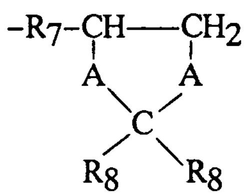

(xi)

où $R_7$ représente un groupe alkylène en $C_1$ à $C_7$, $R_8$ représente un groupe alkyle en $C_1$ à $C_3$ et A a le même sens que celui défini ci-dessus ;

$-CO-R_6$ (xii)

où $R_6$ représente un groupe alkyle en $C_1$ à $C_5$ ;
Y et Z peuvent former ensemble $=CF-CF_3$ ou $=CHOR_6$, formule dans laquelle $R_6$ a le même sens que celui défini ci-dessus ;
à la condition qu'au moins l'un des substituants X, Y et Z soit un atome de fluor ou un groupe contenant du fluor ; lorsque Y est un atome de fluor et que n vaut 0 ou 1, au moins l'un des symboles X et Z ne représente pas un atome d'hydrogène ; lorsque n est nul, le groupe -CXYZ n'est pas un groupe difluorométhyle ; et à la condition que, lorsque n vaut 1 et X et Y représentent respectivement H et OH, le symbole Z ne représentent pas $-CH_2F$,
avec n'importe quel excipient ou véhicule que l'on utilise traditionnellement dans ce domaine et en formulant par une méthode traditionnelle.

Fig. 1

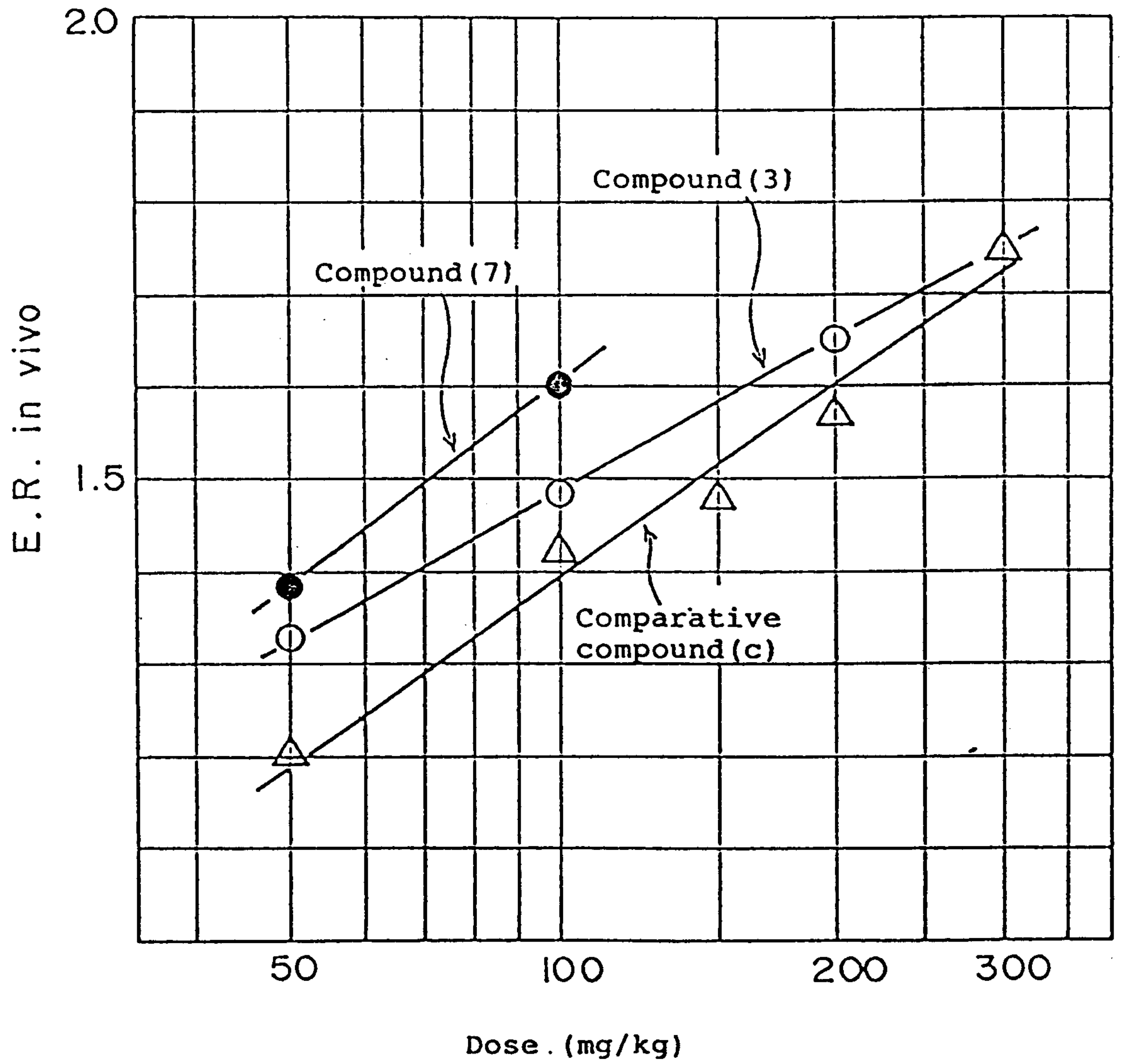
2.0
1.5
E.R. in vivo
Compound(3)
Compound(7)
Comparative compound(c)
50
100
200
300
Dose.(mg/kg)

Fig. 2

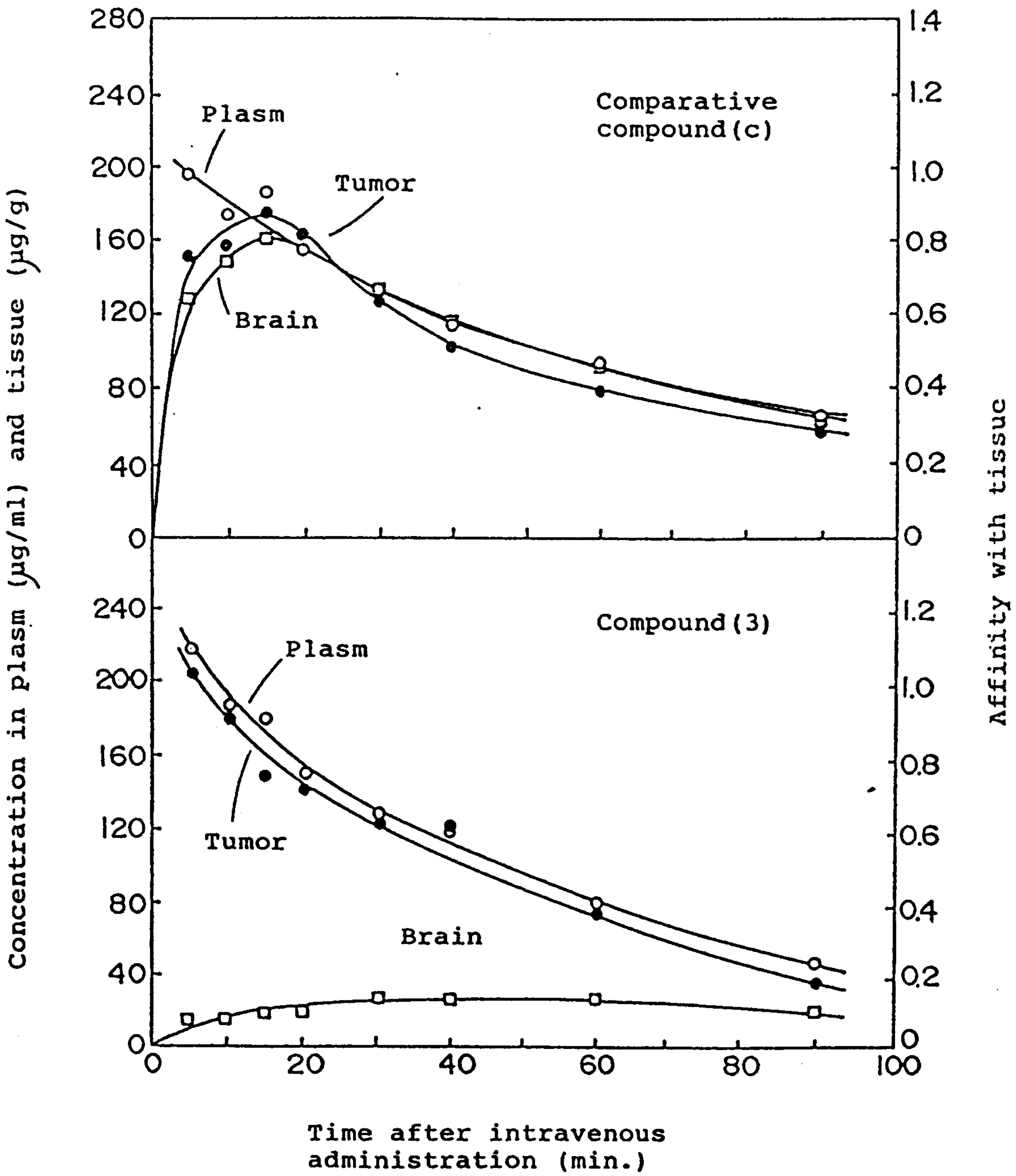

Concentration in plasm (µg/ml) and tissue (µg/g)
Affinity with tissue
Comparative compound (c)
Plasm
Tumor
Brain
Compound (3)
Plasm
Tumor
Brain
280
240
200
160
120
80
40
0
1.4
1.2
1.0
0.8
0.6
0.4
0.2
0
20
40
60
80
100
Time after intravenous administration (min.)